# EUROPEAN PATENT SPECIFICATION

(11) **EP 1 129 724 B1**
(45) Date of publication and mention of the grant of the patent: **19.09.2007**
(21) Application number: 01102609.3
(22) Date of filing: 30.09.1992
(51) Int. Cl.: A61K 39/385

(54) **Vaccines for treating colon cancer**
Impfstoffe zur Behandlung von Kolonkrebs
Vaccins pour traiter le cancer du colon

(43) Date of publication of application: 05.09.2001
(62) Divisional of application: 92921753.7
(73) Proprietor: THE OHIO STATE UNIVERSITY RESEARCH FOUNDATION, Columbus, Ohio 43210-1063 (US)
(72) Inventor: Stevens, Vernon C., Dublin, OH 43017 (US)
(74) Representative: Grund, Martin

(56) References cited:
- EP-A- 0 427 347
- US-A- 4 762 913
- US-A- 5 006 334
- HO P C ET AL: "IDENTIFICATION OF TWO PROMISCUOUS T CELL EPITOPES FROM TETANUS TOXIN" EUROPEAN JOURNAL OF IMMUNOLOGY, vol. 20, no. 3, 1990, pages 477-484, XP001024292 ISSN: 0014-2980
- BAGSHAWE K.D. ET AL: 'A CYTOTOXIC AGENT CAN BE GENERATED SELECTIVELY AT CANCER SITES' BRITISH JOURNAL OF CANCER vol. 58, 1988, LONDON, GB, pages 700 - 703, XP000121545
- PUISIEUX A. ET AL: ENDOCRINOLOGY, vol. 126, no. 2, February 1990 (1990-02), pages 687-694, XP009066365
- CHANG C. ET AL: FERTILITY AND STERILITY, vol. 36, no. 5, November 1981 (1981-11), pages 659-663, XP009066366

### SEQUENCE LISTING

<110> The Ohio State University Research Foundation
<120> Vaccines
<130> 130-11Divl
<140>
   <141> 1992-09-30
<150> EP92921753.7
   <151> 1992-09-30
<150> PCT/US92/08370
   <151> 1992-09-30
<160> 22
<170> PatentIn Ver. 2.1
<210> 1
   <211> 145
   <212> PRT
   <213> Artificial Sequence
<220>
   <223> Description of Artificial Sequence:Antigenic polypeptide
<400> 1
   Ser Lys Glu Pro Leu Arg Pro Arg Cys Arg Pro Ile Asn Ala Thr Leu
    1               5                   10                  15
   Ala Val Glu Lys Glu Gly Cys Pro Val Cys Ile Thr Val Asn Thr Thr
               20                  25                  30
   Ile Cys Ala Gly Tyr Cys Pro Thr Met Thr Arg Val Leu Gln Gly Val
           35                  40                  45
   Leu Pro Ala Leu Pro Gln Val Val Cys Asn Tyr Arg Asp Val Arg Phe
       50                  55                  60
   Glu Ser Ile Arg Leu Pro Gly Cys Pro Arg Gly Val Asn Pro Val Val
   65                  70                  75                  80
   Ser Tyr Ala Val Ala Leu Ser Cys Gln Cys Ala Leu Cys Arg Arg Ser
                   85                  90                  95
   Thr Thr Asp Cys Gly Gly Pro Lys Asp His Pro Leu Thr Cys Asp Asp
               100                 105                 110
   Pro Arg Phe Gln Asp Ser Ser Ser Ser Lys Ala Pro Pro Pro Ser Leu
           115                 120                 125
   Pro Ser Pro Ser Arg Leu Pro Gly Pro Ser Asp Thr Pro Ile Leu Pro
       130                 135                 140
   Gln
   145
<210> 2
   <211> 35
   <212> PRT
   <213> Artificial Sequence
<220>
   <223> Description of Artificial Sequence:Antigenic polypeptide
<400> 2
   Asp Asp Pro Arg Phe Gln Asp Ser Ser Ser Ser Lys Ala Pro Pro Pro
    1               5                   10                  15
   Ser Leu Pro Ser Pro Ser Arg Leu Pro Gly Pro Ser Asp Thr Pro Ile
               20                  25                  30
   Leu Pro Gln
           35
<210> 3
   <211> 29
   <212> PRT
   <213> Artificial Sequence
<220>
   <223> Description of Artificial Sequence:Antigenic polypeptide
<400> 3
   Gln Asp Ser Ser Ser Ser Lys Ala Pro Pro Pro Ser Leu Pro Ser Pro
    1              5                   10                  15
   Ser Arg Leu Gly Pro Ser Asp Thr Pro Ile Leu Pro Gln
               20                  25
<210> 4
   <211> 15
   <212> PRT
   <213> Artificial Sequence
<220>
   <223> Description of Artificial Sequence:Antigenic polypeptide
<400> 4
   Cys Pro Pro Pro Pro Pro Pro Ser Asp Thr Pro Ile Leu Pro Gln
    1              5                   10                  15
<210> 5
   <211> 15
   <212> PRT
   <213> Artificial Sequence
<220>
   <223> Description of Artificial Sequence:Antigenic polypeptide
<400> 5
   Asp Asp Pro Arg Phe Gln Asp Ser Pro Pro Pro Pro Pro Pro Cys
    1              5                   10                  15
<210> 6
   <211> 31
   <212> PRT
   <213> Artificial Sequence
<220>
   <223> Description of Artificial Sequence : Antigenic polypeptide
<400> 6
   Phe Gln Asp Ser Ser Ser Ser Lys Ala Pro Pro Pro Ser Leu Pro Ser
    1              5                   10                  15
   Pro Ser Arg Leu Pro Gly Pro Ser Asp Thr Pro Ile Leu Pro Gln
               20                  25                  30
<210> 7
   <211> 20
   <212> PRT
   <213> Artificial Sequence
<220>
   <223> Description of Artificial Sequence : Antigenic polypeptide
<400> 7
   Asp Asp Pro Arg Phe Gln Asp Ser Ser Ser Ser Lys Ala Pro Pro Pro
    1               5                  10                  15
   Ser Leu Pro Ser
               20
<210> 8
   <211> 23
   <212> PRT
   <213> Artificial Sequence
<220>
   <223> Description of Artificial Sequence:Antigenic polypeptide
<400> 8
   Asp Asp Pro Arg Phe Gln Asp Ser Pro Pro Pro Cys Pro Pro Pro Ser
    1               5                  10                  15
   Asp Thr Pro Ile Leu Pro Gln
               20
<210> 9
   <211> 29
   <212> PRT
   <213> Artificial Sequence
<220>
   <223> Description of Artificial Sequence : Antigenic polypeptide
<400> 9
   Asp Asp Pro Arg Phe Gln Asp Ser Pro Pro Pro Pro Pro Pro Cys Pro
    1               5                  10                  15
   Pro Pro Pro Pro Pro Ser Asp Thr Pro Ile Leu Pro Gln
               20                  25
<210> 10
   <211> 48
   <212> PRT
   <213> Artificial Sequence
<220>
   <223> Description of Artificial Sequence:Antigenic polypeptide
<400> 10
   Asp His Pro Leu Thr Ala Asp Asp Pro Arg Phe Gln Asp Ser Ser Ser
    1               5                  10                  15
   Ser Lys Ala Pro Pro Pro Ser Leu Pro Ser Pro Ser Arg Leu Pro Gly
               20                  25                  30
   Pro Ser Asp Thr Pro Ile Leu Pro Gln Pro Pro Pro Pro Pro Pro Cys
           35                  40                  45
<210> 11
   <211> 42
   <212> PRT
   <213> Artificial Sequence
<220>
   <223> Description of Artificial Sequence : Antigenic polypeptide
<400> 11
   Asp Asp Pro Arg Phe Gln Asp Ser Ser Ser Ser Lys Ala Pro Pro Pro
    1               5                  10                  15
   Ser Leu Pro Ser Pro Ser Arg Leu Pro Gly Pro Ser Asp Thr Pro Ile
                20                  25                  30
   Leu Pro Gln Pro Pro Pro Pro Pro Pro Cys
           35                  40
<210> 12
   <211> 36
   <212> PRT
   <213> Artificial Sequence
<220>
   <223> Description of Artificial Sequence:Antigenic polypeptide
<400> 12
   Asp Asp Pro Arg Phe Gln Asp Ser Ser Ser Ser Lys Ala Pro Pro Pro
    1               5                  10                  15
   Ser Leu Pro Ser Pro Ser Arg Leu Pro Gly Pro Ser Asp Thr Pro Ile
               20                  25                  30
   Leu Pro Gln Cys
           35
<210> 13
   <211> 37
   <212> PRT
   <213> Artificial Sequence
<220>
   <223> Description of Artificial Sequence:Antigenic polypeptide
<400> 13
   Thr Cys Asp Asp Pro Arg Phe Gln Asp Ser Ser Ser Ser Lys Ala Pro
   1               5                   10                  15
   Pro Pro Ser Leu Pro Ser Pro Ser Arg Leu Pro Gly Pro Ser Asp Thr
               20                  25                  30
   Pro Ile Leu Pro Gln
           35
<210> 14
   <211> 42
   <212> PRT
   <213> Artificial Sequence
<220>
   <223> Description of Artificial Sequence:Antigenic polypeptide, Xaa is a symbol for alpha amino butyric acid of cysteine
<400> 14
   Asp His Pro Leu Thr Xaa Asp Asp Pro Arg Phe Gln Asp Ser Ser Ser
    1               5                  10                  15
   Ser Lys Ala Pro Pro Pro Ser Leu Pro Ser Pro Ser Arg Leu Pro Gly
               20                  25                  30
   Pro Ser Asp Thr Pro Ile Leu Pro Gln Cys
           35                  40
<210> 15
   <211> 43
   <212> PRT
   <213> Artificial Sequence
<220>
   <223> Description of Artificial Sequence : Antigenic polypeptide
<400> 15
   Cys Pro Pro Pro Pro Pro Pro Pro Asp Asp Pro Arg Phe Gln Asp Ser
   1 5 10 15
   Ser Ser Ser Lys Ala Pro Pro Pro Ser Leu Pro Ser Pro Ser Arg Leu
   20 25 30
   Pro Gly Pro Ser Asp Thr Pro Ile Leu Pro Gln
   35 40
<210> 16
   <211> 20
   <212> PRT
   <213> Artificial Sequence
<220>
   <223> Description of Artificial Sequence:Antigenic polypeptide
<400> 16
   Cys Pro Ser Met Lys Arg Val Leu Pro Val Ile Leu Pro Pro Met Pro
   1 5 10 15
   Gln Arg Val Cys
   20
<210> 17
   <211> 20
   <212> PRT
   <213> Artificial Sequence
<220>
   <223> Description of Artificial Sequence:Antigenic polypeptide
<400> 17
   Cys Pro Thr Met Met Arg Val Leu Gln Ala Val Leu Pro Pro Leu Pro
   1 5 10 15
   Gln Val Val Cys
   20
<210> 18
   <211> 20
   <212> PRT
   <213> Artificial Sequence
<220>
   <223> Description of Artificial Sequence:Antigenic polypeptide
<400> 18
   Cys Pro Thr Met Thr Arg Val Leu Gln Gly Val Leu Pro Ala Leu Pro
   1 5 10 15
   Gln Val Val Cys
   20
<210> 19
   <211> 20
   <212> PRT
   <213> Artificial Sequence
<220>
   <223> Description of Artificial Sequence:Antigenic polypeptide
<400> 19
   Cys Tyr Thr Arg Asp Leu Val Tyr Lys Asn Pro Ala Arg Pro Lys Ile
   1 5 10 15
   Gln Lys Thr Cys
   20
<210> 20
   <211> 20
   <212> PRT
   <213> Artificial Sequence
<220>
   <223> Description of Artificial Sequence:Antigenic polypeptide
<400> 20
   Cys Tyr Thr Arg Asp Leu Val Tyr Lys Asp Pro Ala Arg Pro Lys Ile
   1 5 10 15
   Gln Lys Thr Cys
   20
<210> 21
   <211> 20
   <212> PRT
   <213> Artificial Sequence
<220>
   <223> Description of Artificial Sequence:Antigenic polypeptide
<400> 21
   Cys Pro Ser Met Val Arg Val Thr Pro Ala Ala Leu Pro Ala Ile Pro
   1 5 10 15
   Gln Pro Val Cys
   20
<210> 22
   <211> 22
   <212> PRT
   <213> Artificial Sequence
<220>
   <223> Description of Artificial Sequence:Antigenic polypeptide
<400> 22
   Cys Met Thr Arg Asp Ile Asp Gly Lys Leu Phe Leu Pro Lys Tyr Ala
    1               5                  10                  15
   Leu Ser Gln Asp Val Cys
               20

## Description

This invention relates to a vaccine comprising an antigenic conjugates of a protein reproductive hormone, and an epitope peptide.

In the present inventor's United States Patents Nos. 4,201,770; 4,302,386; 4,384,995; 4,526,716; 4,691,006; 4,713,366; 4,855,285; and 5,006,334; in his International Patent Application WO 84/03443 ; and in his numerous corresponding patents and PUBLISHED applications in other countries, there are described and claimed antigenic polypeptides which are obtained by coupling a female protein reproductive hormone, a fragment of such a hormone, or a peptide substantially immunologically equivalent to such a hormone or fragment, to a non-endogenous material having a size sufficient to elicit antibody response following the administration thereof into the body of a human or other mammal. When administered to humans or other mammals, these antigenic polypeptides cause the development of antibodies to the female protein reproductive hormone from which they are derived, and the antigenic polypeptides are thus useful for controlling biological activity in humans or other mammals. The biological activity controlled can be, inter alia, fertility or the development of malignant tumors. In a preferred form of such antigenic polypeptides, the carrier is diphtheria toxoid. The aforementioned patents and applications also disclose vaccines containing the antigenic polypeptides together with an adjuvant and an oil, the antigenic polypeptide and the adjuvant being dispersed in an aqueous medium to form an aqueous phase and this aqueous phase being emulsified with the oil.

This invention provides an improved form of the vaccine described in the aforementioned patents and applications.

In one aspect, this invention provides a vaccine AS DEFINED IN CLAIM 1.

This invention also provides the use of such a vaccine as defined in claim 11.
Figure 1 shows the formulae of three coupling agents used to prepare the conjugates of the present invention; and
Figure 2 shows a typical reaction used to prepare conjugates of the present invention, together with examples of the modified polypeptides produced by such conjugation reactions.

As already mentioned, the present invention provides a vaccine comprising the antigenic conjugate of a protein reproductive hormone conjugated with a chemically-modified diphtheria toxoid AS DEFINED IN CLAIM 1. The vaccine may also comprise an adjuvant and a mixture of oils. To form the vaccine, the conjugate and adjuvant are dispersed in an aqueous medium, preferably phosphate-buffered saline, to form an aqueous phase and this aqueous phase is emulsified with the mixture of oils.

As compared with the inventor's earlier vaccines described in the aforementioned patents and applications, the vaccine of the present invention exhibits an improved ability to attract immune cells to the injection site, and thus to generate the desired antibody response from the human or other animal being treated. Preferred embodiments of the present vaccine also form a thick emulsion which slowly releases the antigenic conjugate from the injection site, thus providing a desirable long term development of antigenic response.

Diphtheria toxoid suitable for use in the vaccines of the present invention is available commercially, for example from Connaught Laboratories, Swiftwater, Massachusetts; such diphtheria toxoid may be reacted with ethylenediamine as described below. The ratio of reproductive hormone, fragment or peptide to conjugate in the vaccine can vary widely, but desirably, the antigenic conjugate comprises 20-30 peptides per 10⁵ daltons of the chemically-modified diphtheria toxoid.

A preferred adjuvant for use in the vaccines of the present invention is N-acetyl-D-glucosamine-3-yl-acetyl-L-ala-D-isoglutamine (nor muramyl dipeptide), while a preferred mixture of oils comprises squalene and squalane, desirably containing one or both of mannide monooleate and dissolved or suspended aluminum monostearate. Preferably, such a mixture comprises, by weight, from 35 to 45 percent of squalene, from 35 to 45 percent of squalane, from 6 to 16 percent of mannide monooleate and from 1 to 5 percent of aluminum monostearate, with a specific preferred formulation being 44 percent squalene, 41 percent squalane, 11 percent mannide monooleate and 4 percent dissolved or suspended aluminum monostearate. Although the ratio of the various constituents of the vaccine can vary widely, desirably, from about 0.5 to about 2.0 mg of the antigenic conjugate and from about 0.2 to about 1.0 mg of the adjuvant are present per ml of the final emulsion. Conveniently, the vaccine comprises substantially equal volumes of the aqueous phase and the mixture of oils.

The vaccines of the present invention can be used for the treatment of humans (or other animals) suffering from colon cancer.

The vaccines of the present invention have been found effective in attracting immune cells to the site of the injection, while the physical nature of the vaccine, which is a thick emulsion, also helps to ensure a slow release of the conjugate.

Also as already mentioned, the vaccine of the invention employs an antigenic conjugate of a protein reproductive hormone, a fragment of such a hormone, or a peptide substantially immunologically equivalent to such a hormone or fragment coupled, at either its N-terminal or its C-terminal, to an epitope peptide having the sequence of at least one foreign T cell lymphocyte epitope, or a sequence substantially immunologically equivalent thereto. Conjugates formed with T cell epitopes tend to be cheaper to produce than the conjugates formed with complex carriers such as diphtheria toxoids described in the aforementioned patents and applications. Furthermore, because the T cell epitopes are much simpler in structure than diphtheria toxoid and similar complex carriers, T cell epitope conjugates are less likely to provoke hypersensitivity reactions. Until recently, the use of T cell epitopes in conjugates was counterindicated because genetic variations in outbred populations of humans or other animals responded to different T cell epitopes on foreign molecules, and when only a single T cell epitope was provided (as would normally be the case when using an antigenic conjugate) not all of the animals treated gave positive responses, thus rendering the T cell epitope conjugate unreliable. However, recently certain T cell epitopes from foreign molecules have been shown not to be limited by such genetic restrictions, and the use of these T cell epitopes enables the preparation of conjugates and vaccines which give reliable antibody responses in genetically-diverse populations of humans and other animals. See, for example, Ho et al., Eur. J. Immunol., 20, 477-483 (1990), and Partidos et al., J. Gen. Virology, 71, 2099-2105 (1990).

The epitode peptides for use in the vaccines of the present invention are those having a sequence corresponding to
a. amino acids 580-599 of tetanus toxoid:

   Asn-Ser-Val-Asp-Asp-Ala-Leu-Ile-Asn-Ser-Thr-Lys-Ile-Try-Ser-Tyr-Phe-Pro-Ser-Val
b. amino acids 830-844 of tetanus toxoid

   Gln-Try-Ile-Lys-Ala-Asn-Ser-Lys-Phe-Ile-Gly-Ile-Thr-Glu-Leu
c. amino acids 916-932 of tetanus toxoid:

   Pro-Gly-Ile-Asn-Gly-Lys-Ala-Ile-His-Leu-Val-Asn-Asn-Gln-Ser-Ser-Glu
d. amino acids 947-967 of tetanus toxoid:

   Phe-Asn-Asn-Phe-Thr-Val-Ser-Phe-Trp-Leu-Arg-Val-Pro-Lys-Val-Ser-Ala-Ser-His-Leu-Glu
e. amino acids 288-302 of measles virus protein:

   Leu-Ser-Glu-Ile-Lys-Gly-Val-Ile-Val-His-Arg-Leu-Glu-Gly-Val
f. amino acids 16-33 of hepatitis B viral protein:

   Gln-Ala-Gly-Phe-Phe-Leu-Leu-Thr-Arg-De-Leu-Thr-Ile-Pro-Gln-Ser-Leu-Asp

   or
g. amino acids 317-336 of malaria CSP protein:

   Thr-Cys-Gly-Val-Gly-Val-Arg-Val-Arg-Ser-Arg-Val-Asn-Ala-Ala-Asn-Lys-Lys-Pro-Glu.
As discussed in more detail below, the epitope peptide is coupled via a spacer peptide, this spacer peptide containing from about 2 to about 8 amino acid residues to the fragment used to form the conjugate, or the coupling may be effected.

Like the vaccines of the present invention the T cell epitope conjugates can be used for any of the purposes described in the aforementioned patents and applications, including fertility control, or the treatment of humans (or other animals) suffering from colon cancer. In some cases, it may be advantageous to use a mixture of two or more T cell epitope conjugates for this purpose in order to secure improved antibody production.

Many of the preferred features of the vaccines of the present invention are generally similar to those described in the aforementioned patents and applications.

The protein reproductive hormone, a fragment of such a hormone, or a peptide substantially immunologically equivalent to such a hormone or fragment may be of natural or synthetic origin. A synthetic hormone molecule will perform the same function as the naturally occurring one, being equivalent for the purpose of this invention. In this connection, it will be noted that certain natural substances with which this invention is concerned possess carbohydrate moieties attached at certain sites thereon whereas the corresponding synthetic polypeptides do not. Nevertheless, for the purpose of the instant specification and claims, the synthetic and natural polypeptides are treated as equivalents and both are intended to be embraced by this invention.

Thus, where the word "hormone" or "hormone molecule" is used herein, the word "synthetic" may be added before "hormone" without changing the meaning of the discussion. Similarly, the word "fragment" may be inserted after "hormone" or "molecule" without changing the meaning, whether or not "synthetic" has been inserted before "hormone".

The term "endogenous" is used herein to denote a protein which is native to the species to be treated, regardless of whether the relevant protein, fragment or antigen is endogenous to the particular individual animal being treated. Thus, for example, for purposes of this application, a porcine sperm antigen is regarded as being endogenous to a sow even though obviously such a sperm antigen will not normally be present in the body of a sow. Similarly, an embryonic, fetal or placental antigen of an animal is regarded as being endogenous to an adult animal of the same species even though such antigens may not exist in the body of the animals after birth. Further antigens produced from an animal's normal cells that have been transformed by mutagenous or other genetic deviation should be considered endogenous to the species in which those cells reside at the time of transformation or deviation.

The antigenic conjugates, which are derived from endogenous protein reproductive hormones, fragments thereof, or peptides equivalent thereto, provoke, when administered into the bodies of appropriate mammals, antibodies to the endogenous proteins from which the modified polypeptides are derived, and the formation of lymphoma cells capable of expressing such antibodies. Consequently, not only can such conjugates be used to influence the biological activity in a mammal to which they are administered by generating antibodies to an endogenous protein in the mammal, but the conjugates of the invention can also be used to generate antisera and/or lymphoma cells by introducing the conjugates into the body of a mammal, thereby provoking the formation, in the mammal, of antibodies to the "endogenous protein"; note that in such a method, since the conjugate need not be introduced into the same mammal, or even a mammal of the same species, as the animal from which it is derived or, in the case of a conjugate based upon a synthetic fragment, the mammal whose protein it mimics, the so-called "endogenous protein" used in this method need not be endogenous to the mammal in which the antibodies are raised.

Following the raising of the antibodies and/or lymphoma cells in the mammal, some of the antibodies and/or cells may be recovered from the mammal, using conventional techniques which will be familiar to those skilled in the art of immunology. Techniques generating monoclonal antibodies may also be used to generate the desired antibodies; for example, lymphoma cells generated as described above may be used to form hybridoma cells capable of expressing the relevant antibodies by conventional techniques which will be known to those skilled in hybridoma technology. The antibodies thus generated can then be used for a variety of purposes. For example, such antibodies may be used for assaying the quantity of an endogenous protein in a mammal by bringing at least some of the recovered antibodies into contact with body tissue or body fluid from the mammal and observing the formation or non-formation of the reaction process between the recovered antibody and the endogenous protein indicative of the presence or absence of the endogenous protein in the body tissue or body fluid assayed. If, in this method, the endogenous protein assayed is one associated with pregnancy, this assay method can function as a pregnancy test. If, on the other hand, the endogenous protein assayed is one the presence or absence of which is associated with reduced fertility or infertility in the mammal from which the body tissue or body fluid is derived, the assay can function as a test for reduced fertility or infertility in such a mammal.

### Selection of Hormone, Fragment or Peptide for Modification

Examples of natural protein reproductive hormones which may be modified according to this invention include Follicle Stimulating Hormone (FSH), Luteinizing Hormone (LH), Luteinizing Hormone Releasing Hormone (LH-RH), relaxin, Chorionic Gonadotropin (CG), e.g. Human Chorionic Gonadotropin (HCG), Placental Lactogen, e.g. Human Placental Lactogen (HPL) and Prolactin, e.g. Human Prolactin. There are certain considerations which should always be borne in mind when considering the selection of an appropriate polypeptide for modification. Firstly, it is of course necessary to determine which hormone or combination of hormones or other protein is responsible for the condition or problem which it is desired to treat. However, in many cases this will still leave one with a large number of possible proteins which could be modified. For example; if one wishes to provide a conjugate to render a female mammal infertile, one can approach the problem by modifying FSH, LH, LH-RH, CG; PL, relaxin or other protein hormones which known to be involved in the female mammalian reproductive system. One important consideration which should always be borne in mind in choosing a polypeptide for modification is the problem of cross-reactivity. As well known to those skilled in the field of immunology, it is not uncommon to find that antibodies intended to react with one protein (the "target" protein) also react to a significant extent with other, non-target proteins. This is a serious problem, since it may cause the administration of a conjugate intended to provoke the formation of antibodies to one specific natural hormone to cause the generation of antibodies to one or more other hormones, which it is not desired to effect. In some cases, the reactions with the non-target proteins may cause damage to essential body functions. Accordingly, so far as possible the hormone, fragment or peptide selected for modification should be chosen so that the conjugate will provoke, in the body of the mammal to be treated, the formation of antibodies which are highly specific to the target protein. In some cases, especially where the target protein is relatively small (for example LH-RH), it may be in practice essential to modify the whole target protein, since a fragment comprising less than the whole target protein, will, even when modified by the instant techniques, fail to provoke sufficient antibodies to the target protein. However, in general, especially when dealing with relatively complex target proteins such as HCG, the use of a fragment of the target protein rather than the intact target protein is recommended for use in forming a conjugate. It is well recognized by those skilled in immunology (see e.g. W. R Jones, "Immunological Fertility Regulation", Blackwell Scientific Publications, Victoria, Australia (1982) pages 11 et. seq.,) that one of the greatest potential hazards of a vaccine, especially a contraceptive vaccine, is cross-reactivity with non-target antigens, producing what is essentially an artificially-induced autoimmune disease capable of causing immunopathological lesions in, and/or loss of function of, the tissues carrying the cross-reactive antigens. Two possible mechanisms for such cross-reactivity are:
(a) presence of shared antigenic determinants; a complex target protein may contain components (amino-acid sequences) identical to those present in non-target proteins; and
(b) steric overlap between non-identical but structurally related parts of the target and non-target proteins.

Obviously, the threats posed by both these modes of cross-reactivity may be lessened by using a fragment of a complex protein rather than the whole protein. Since the fragment has a simpler structure than the protein from which it is derived, there is less chance of shared antigenic determinants or steric overlap with non-target proteins. In particular, cross-reactions can be avoided by using fragments derived from a portion of the target protein which is not similar in sequence to the non-target but cross-reactive protein. To take one specific example, one of the major problems in provoking antibodies to HCG is cross-reactivity of HCG antibodies with LH, this cross-reactivity being at least largely due to virtual identity of amino acid sequence between LH and the 1-110 amino acid sequence of the beta subunit of HCG. Accordingly, when it is desired to form an HCG-derived conjugate of the invention, the fragment used is preferably one having a molecular structure similar to part or all of the 111-145 (or, for reasons discussed below, the 109-145) sequence of the beta subunit of HCG, since it is only this 111-145 sequence of beta-HCG which differs significantly from the corresponding sequence of LH. However, as discussed in more detail below, fragments of mammalian luteinizing hormones, chorionic gonadotropins or follicle secreting hormones having amino acid sequences resembling the 38-57 region of the beta-subunit or human chorionic gonadotropin are also useful in the present invention.

Thus, in most cases the polypeptide modified is preferably a fragment of the target protein rather than the intact target protein. More accurately, one should use, as the fragment of a polypeptide to be modified, a fragment which has a molecular structure similar to a fragment of the target protein. In saying that the fragment has a molecular structure similar to a fragment of the target protein, it is not necessarily implied that the entire amino acid sequence of the fragment must correspond exactly to part of the sequence of the target protein. For example, in certain cases some substitution of amino acids may be possible without effecting the immunogenic character of the fragment See the aforementioned U.S. Patent No. 4,302,386, which describes a polypeptide, designated Structure (IX) (which is also discussed in detail below), which is notionally derived from the beta subunit of HCG but in which the cysteine residue at the 110-position is replaced by alpha-aminobutyric acid. Furthermore, although the natural form of the beta subunit of HCG contains a number of carbohydrate residues attached to the amino-acid chain, synthetic peptides corresponding in sequence to the relevant parts of the HCG sequence, but lacking such carbohydrate residues, can be modified to give good results.

Of course, one should be cautious when using sequences not exactly corresponding to portions of the target protein. For example, the protein relaxin is known to be highly species specific and accordingly it is not recommended that fragments of non-human relaxin proteins be modified by the instant methods and injected into humans to provoke the formation of anti-relaxin-antibodies in humans. In choosing an appropriate hormone, fragment or peptide for formation of a conjugate, amino-acid sequence is, however, not the only factor which has to be considered; it is also necessary to pay close attention to the conformation, that is to say the physical shape, of the protein, fragment or peptide selected relative to the natural conformation of the target protein. It is well known to those skilled in the art of immunology that the conformation or shape of an antigen is an important factor in allowing recognition of the antigen by an antibody. Accordingly, if a conjugate does not retain the conformation of the relevant part of the target protein, it is likely that the antibodies provoked by injection of the conjugate into a mammal will not display optimum activity against the natural target protein. For example, a peptide having the same sequence as part of the target protein will probably not work very well if, because of the absence of other parts of the sequence of the target protein which affect the positioning of the crucial antigenic determinant in the natural target protein, the fragment used to prepare the conjugate adopts a conformation very different from the conformation of the same amino acid sequence in the target protein. Similarly, because of the way in which the chain of a complex target protein will normally be folded, the antigen-antibody binding reaction may rely upon recognition of two or more amino acid sequences which are widely separated along the chain of the target protein but lie, in the natural conformation of the target protein, closely adjacent one another in space.

As those skilled in the art are aware, one major factor effecting the conformation, and hence the antigenic properties and antigenic determinants, of complex proteins is the presence of cysteine residues and disulfide bridges in such proteins. It is well know to those skilled in the art that, in many natural proteins containing cysteine residues, these residues are not present in their thiol form containing a free -SH group. Instead, pairs of cysteine residues are linked by means of disulfide bridges to form cystine. Such disulfide bridges are very important in determining the conformation of the protein. In most cases, the disulfide bridges present in the natural form of the protein are easily reduced to thiol groups by means of mild reducing agents under conditions which leave the remaining parts of the protein molecule unchanged. Such breaking of disulfide bridges causes major changes in the conformation of the protein even though no disturbance of the amino acid sequence occurs. In particular, the twelve cysteine residues present in the beta subunit of HCG are, in the natural form of the subunit, coupled together to form six disulfide bridges, so that the natural form of the protein has no free thiol groups.

The generation of free thiol groups by reduction of disulfide bridges in naturally occurring forms of proteins may affect the cross-reactivity of the antibodies produced when a conjugate derived from the protein or a fragment thereof is injected into an animal. As already mentioned, an antibody frequently recognizes its corresponding antigen not only by the amino acid sequence in the antigen but also by the conformation of the antigen. Accordingly, an antibody which binds very strongly to a protein or a peptide in its natural conformation may bind much less strongly, if at all, to the same protein or polypeptide after its conformation has been drastically altered by breaking disulfide bridges therein.

Accordingly, the breaking of disulfide bridges in proteins or other polypeptides may provide a basis for reducing the cross-reactivity between antibodies to antigens having the same amino acid sequence along parts of the molecule. For example, it has been pointed out above that cross-reaction is frequently encountered between antibodies to beta-HCG and HLH because the first 110 residues in the beta-HCG and HLH sequence are virtually identical in the natural forms of the two molecules, thus the conformations are also presumably very similar. It has been suggested above that one means of producing antibodies to beta-HCG in an animal which do not substantially cross-react with HLH is to supply to the animal a conjugate derived from a polypeptide which contains all or part of the residues 111-145 of beta-HCG but which lacks all or substantially all of the residues 1-110 of beta-HCG. In effect, this approach avoids antibody cross-reaction with HLH by chemically removing from the conjugate the sequence of residues which is common to beta-HCG and HLH. As an alternative approach, by cleaving the appropriate number of disulfide bridges in the natural form of beta-HCG, it may be possible to so alter the conformation of residues 1-110 thereof that the antibodies formed when a conjugate based upon this altered-conformation beta-HCG is administered to an animal will no longer cross-react significantly with HLH. In other words, instead of chemically severing the common sequence of residues from beta-HCG in order to prevent cross-reaction, it may be possible to leave this common sequence of residues in the beta-HCG but to so alter the conformation of this common sequence that, to an antibody, the altered-conformation common sequence does not "look" like the natural form of the common sequence, so that an antibody which recognizes the altered-conformation common sequence will not recognize the natural-conformation common sequence in HLH. Moreover, once the natural conformation of the sequence of residues 1-110 has been destroyed by breaking the disulfide bridges, this common sequence will probably assume the helical conformation common in polypeptides lacking disulfide bridges, so that this part of the beta-HCG will not be strongly immunogenic and most of the antibodies formed by a conjugate based upon the altered-conformation beta-HCG will be antibodies to the sequence 111-145 which is not common with HLH. Obviously, cross-reactivity between antibodies to other pairs of hormones may similarly be destroyed by altering the conformation of portions of the two proteins which are similar and hence will otherwise promote antigen cross-reactivity.

### Chorionic Gonadotropin and Related Hormones

The hormone, Chorionic Gonadotropin (CG) has been the subject of extensive investigation, it being demonstrated in 1927 that the blood and urine of pregnant women contained a gonad-stimulating substance which, when injected into laboratory animals, produced marked gonadal growth. Later, investigators demonstrated with certainty that the Placental Chorionic villi, as opposed to the pituitary, were the source of this hormone. Thus, the name Chorionic Gonadotropin or, in the case of humans, Human Chorionic Gonadotropin (HCG) was given to this hormone of pregnancy. During the more recent past, a broadened variety of studies have been conducted to describe levels of HCG in normal and abnormal physiological states, indicating its role in maintaining pregnancy. The studies have shown the hormone's ability to induce ovulation and to stimulate corpus luteum function, and evidence has been evoked for showing its ability to suppress lymphocyte action. The immunological properties of the HCG molecule also have been studied widely. Cross-reaction of antibodies to HCG with human pituitary Luteinizing Hormone (LH), and vice-versa, has been extensively documented, see for example:
Paul, W. E. & Ross, F. T., Immunologic Cross Reaction Between HCG and Human Pituitary Gonadotropin. Endocrinology, 75, 352-358 (1964);
Flux, D. X. & Li C. H., Immunological Cross Reaction Among Gonadotropins. Acta Endocrinologic, 48, 61-72 (1965);
Bagshawe, K. D.; Orr, A. H. & Godden J., Cross-Reaction in Radio-Immunoassay between HCG and Plasma from Various Species. Journal of Endocrinology, 42, 513-518 (1968);
Franchimont, P., Study on the Cross-Reaction between HCG and Pituitary LH. European Journal of Clinical Investigation, 1, 65-68 (1970);
Domer, M.; Brossmer, R; Hilgenfeldt, U. and Trude, E., Immunological reactions of Antibodies to HCG with HCG and its chemical derivatives in Structure-Activity Relationships of Proteins and Polypeptide Hormones (ed. M Margoulies & F. C. Greenwood), pp 539, 541 Amsterdam: Excerpta Medica Foundation (1972);
Further, these cross-reactions have been used to perform immunoassays for both CG and LH hormones. See:
Midgley, A. R Jr., Radioimmunoassay: a method for HCG and LH. Endocrinology, 79, 10-16 (1966);
Crosignani, P. G., Polvani, F. & Saracci R, Characteristics of a radioimmunoassay for HCG-LH in Protein and Polypeptide Hormones (ed. M. Margoulies) pp. 409, 411 Amsterdam: Excerpta Medica Foundation (1969);
Isojima, S; Nake, O.; Kojama, K.; & Adachi, H. Rapid radioimmunoassay of human L. H. using polymerized antihuman BCG as immunoadsorbent. Journal of Clinical Endocrinology and Metabolism, 31, 693-699 (1970).
As already noted there is a large portion of the beta subunit of CG which is almost identical to the corresponding beta subunit of LH, so that it is desirable to use a fragment corresponding to a portion of the 111-145 sequence of the beta subunit of CG which is not common to LH, thereby avoiding the cross-reactivity of CG and LH antibodies already discussed above. Thus, an immunological reaction against the hormone CG can be achieved without causing undesirable immune reactions to the naturally occurring body constituent LH. Synthetic polypeptides corresponding to the desired fragments of CG offer enhanced practicality both from the standpoint of production costs and the high degree of purity needed for commercial use in a contraceptive agent

Subunits and fragments of the proteinaceous reproductive hormones include the beta subunit of natural Follicle Stimulating Hormone, the beta subunit of natural Human Chorionic Gonadotropin, fragments including, inter alia, a 20-30 or 30-39 amino acid peptide consisting of the C-terminal residues of natural Human Chorionic Gonadotropin beta subunit, as well as specific unique fragments of natural Human Prolactin and natural Human Placental Lactogen, which may bear little resemblance to analogous portions of other protein hormones. Further with respect to the type of novel chemical entities with which this invention is concerned, one may note for instance the chemical configuration of the beta subunit of HCG. That structure is as follows:

| Structure (I) |
|---|
| Ser-Lys-Glu-Pro-Leu-Arg-Pro-Arg-Cys-Arg-Pro- |
| Ile-Asn-Ala-Thr-Leu-Ala-Val-Glu-Lys-Glu-Gly- |
| Cys-Pro-Val-Cys-Ile-Thr-Val-Asn-Thr-Thr-Ile- |
| Cys-Ala-Gly-Try-Cys-Pro-Thr-Met-Thr-Arg-Val- |
| Leu-Gln-Gly-Val-Leu-Pro-Ala-Leu-Pro-Gln-Val- |
| Val-Cys-Asn-Try-Arg-Asp-Val-Arg-Phe-Glu-Ser- |
| Ile-Arg-Leu-Pro-Gly-Cys-Pro-Arg-Gly-Val-Asn- |
| Pro-Val-Val-Ser-Try-Ala-Val-Ala-Leu-Ser-Cys- |
| Gln-Cys-Ala-Leu-Cys-Arg-Arg-Ser-Thr-Thr-Asp- |
| Cys-Gly-Gly-Pro-Lys-Asp-His-Pro-Leu-Thr-Cys- |
| Asp-Asp-Pro-Arg-Phe-Gln-Asp-Ser-Ser-Ser-Ser- |
| Lys-Ala-Pro-Pro-Pro-Ser-Leu-Pro-Ser-Pro-Ser- |
| Arg-Leu-Pro-Gly-Pro-Ser-Asp-Thr-Pro-Ile-Leu- |
| Pro-Gln |

For specificity of antibody action it is necessary that distinctive peptides be isolated or prepared that contain molecular structures completely or substantially completely different from the other hormones. The beta subunit of HCG possesses a specific chain or chains of amino acid moieties which differ either completely or essentially from the polypeptide chain of Human Luteinizing Hormone. These chains or fragments, when conjugated with a carrier, represent an additional aspect of this invention. Accordingly, the polypeptide Structures (II) and (III) [C-terminal portions of structure I]

| Structure (II) |
|---|
| Asp-Asp-Pro-Arg-Phe-Gln-Asp-Ser-Ser-Ser-Ser- |
| Lys-Ala-Pro-Pro-Pro-Ser-Leu-Pro-Ser-Pro-Ser- |
| Arg-Leu-Pro-Gly-Pro-Ser-Asp-Thr-Pro-Ile-Leu- |
| Pro-Gln |

| |
|---|
| Structure (III) |
| Gln-Asp-Ser-Ser-Ser-Ser-Lys-Ala-Pro-Pro-Pro- |
| Ser-Leu-Pro-Ser-Pro-Ser-Arg-Leu-Gly-Pro-Ser- |
| Asp-Thr-Pro-Ile-Leu-Pro-Gln |

whether obtained by purely synthetic methods or by enzymatic degradation from the natural or parent polypeptide, [Carlson et al., J. Biological Chemistry, 284 (19), 6810, (1973)] when modified according to this invention, similarly provide materials with antigenic properties sufficient to provide the desired immunological response.

The beta subunit set forth at structure (I) is seen to represent a chemical sequence of 145 amino acid components. This structure has a high degree of structural homology with the corresponding subunit of Luteinizing Hormone (LH) to the extent of the initial 110 amino acid components. As indicated above, it may be found desirable, therefore to evoke a high specificity to the Chorionic Gonadotropin hormone or an analogous entity through the use of fragments analogous to the C-terminal, 111-145 amino acid sequence of the subunit. Structure (II) above may be observed to represent just that sequence. Structure (III) is slightly shorter, representing the 116-145 amino acid positions within the subunit sequence.

Further polypeptide chains useful in the present antigenic conjugates to promote antibody build-up against natural CG include the following structures labeled Structures (IV)-(XIV). In the present conjugates, these polypeptides provide immunogenic activity against HCG. All of these polypeptides are considered fragments of HCG by virtue of their substantial resemblance to the chemical configuration of the natural hormone and the immunological response provided by them when modified by the instant processes.

| Structure (IV) |
|---|
| Cys-Pro-Pro-Pro-Pro-Pro-Pro-Ser-Asp-Thr-Pro- |
| Ile-Leu-Pro-Gln |

| |
|---|
| Structure (V) |
| Asp-Asp-Pro-Arg-Phe-Gln-Asp-Ser-Pro-Pro-Pro- |
| Pro-Pro-Pro-Cys |

| |
|---|
| Structure (VI) |
| Phe-Gln-Asp-Ser-Ser-Ser-Ser-Lys-Ala-Pro-Pro- |
| Pro-Ser-Leu-Pro-Ser-Pro-Ser-Arg-Leu-Pro-Gly- |
| Pro-Ser-Asp-Thr-Pro-Ile-Leu-Pro-Gln |

| |
|---|
| Structure (VII) |
| Asp-Asp-Pro-Arg-Phe-Gln-Asp-Ser-Ser-Ser-Ser- |
| Lys-Ala-Pro-Pro-Pro-Ser-Leu-Pro-Ser |

| |
|---|
| Structure (VIII) |
| Asp-Asp-Pro-Arg-Phe-Gln-Asp-Ser-Pro-Pro-Pro- |
| Cys-Pro-Pro-Pro-Ser-Asp-Thr-Pro-Ile-Leu-Pro-Gln |

| |
|---|
| Structure (VIIIa) |
| Asp-Asp-Pro-Arg-Phe-Gln-Asp-Ser-Pro-Pro-Pro- |
| Pro-Pro-Pro-Cys-Pro-Pro-Pro-Pro-Pro-Pro-Ser- |
| Asp-Thr-Pro-Ile-Leu-Pro-Gln |

| |
|---|
| Structure (IX) |
| Asp-His-Pro-Leu-Thr-Ala-Asp-Asp-Pro-Arg-Phe- |
| Gln-Asp-Ser.-Ser-Ser-Ser-Lys-Ala-Pro-Pro-Pro- |
| Ser-Leu-Pro-Ser-Pro-Ser-Arg-Leu-Pro-Gly-Pro- |
| Ser-Asp-Thr-Pro-Ile-Leu-Pro-Gin-Pro-Pro-Pro- |
| Pro-Pro-Pro-Cys |

| |
|---|
| Structure (X) |
| Asp-Asp-Pro-Arg-Phe-Gln-Asp-Ser-Ser-Ser-Ser |
| Lys-Ala-Pro-Pro-Pro-Ser-Leu-Pro-Ser-Pro-Ser- |
| Arg-Leu-Pro-Gly-Pro-Ser-Asp-Thr-Pro-Ile-Leu- |
| Pro-Gln-Pro-Pro-Pro-Pro-Pro-Pro-Cys |

| Structure (XI) |
|---|
| Asp-Asp-Pro-Arg-Phe-Gln-Asp-Ser-Ser-Ser-Ser- |
| Lys-Ala-Pro-Pro-Pro-Ser-Leu-Pro-Ser-Pro-Ser- |
| Arg-Leu-Pro-Gly-Pro-Ser-Asp-Thr-Pro-Ile-Leu- |
| Pro-Gln-Cys |

Structure (IV) will be recognized as incorporating a Cys component at the amino or N-terminal which is associated with a proline spacer sequence. These spacers serve to position the sequence which follows physically distant from the carrier-modifier. The latter sequence may be observed to present the 138th to 145th amino acid sequence of the subunit Structure (1). Structure (V) on the other hand, represents an initial sequence corresponding with the 111 th to 118th amino acid sequence of the subunit Structure (I) followed by a sequence of six proline spacer components and a carboxyl terminal, present as cysteine. The rationale in providing such a spacer component is to eliminate sites which may remain antigenically neutral in performance. Structures (IV) and (V) represent relatively shorter amino acid sequences to the extent that each serves to develop one determinant site. Consequently, as alluded to in more detail hereinafter, they are utilized in conjunction with a mixed immunization technique wherein a necessary two distinct determinants are provided by the simultaneous administration of two such fragments, each conjugated separately. Structure (VI) represents the 115th through 145th component, sequence of structure (I). Structure (VII) represents a portion of Structure (I); however, essentially, a sequence of the 111th to 130th components thereof is formed.

Structure (VIII) incorporates two sequences, one which may be recognized in Structure (V) and the other in Structure (IV). These two sequences are separated by two spacer sequences of proline components and one is joined with an intermediately disposed cysteine component which serves a conjugation function. With this arrangement, two distinct determinant sites are developed in physically spaced relationship to avoid the development of an unwanted artificial determinant possibly otherwise evolved in the vicinity of their mutual coupling. Structure (VIIIa) represents Structure (VII) with additional proline spacer residues to provide a widened spacing of determinant sites.

Structure (IX) mimics sequences from Structure (I) with the addition of a proline spacer sequence, a cysteine component at the C-terminal, and an Aba substituted for cysteine at the 110 position. The Aba designation is intended herein to mean alpha aminobutyric acid of Cysteine. Structure (X) will be recognized as a combination of Structure (II) with a six residue proline spacer sequence and a cysteine component at the C-terminal. Similarly, Structure (XI) combines Structure (II) with a cysteine component at the C-terminal without a proline spacer sequence.

Other useful peptides include:

| Structure (XII) |
|---|
| Thr-Cys-Asp-Asp-Pro-Arg-Phe-Gln-Asp-Ser-Ser- |
| Ser-Ser-Lys-Ala-Pro-Pro-Pro-Ser-Leu-Pro-Ser- |
| Pro-Ser-Arg-Leu-Pro-Gly-Pro-Ser-Asp-Thr-Pro- |
| Ile-Leu-Pro-Gln |

| |
|---|
| Structure (XIII) |
| Asp-His-Pro-Leu-Thr-Aba-Asp-Asp-Pro-Arg-Phe- |
| Gln-Asp-Ser-Ser-Ser-Ser-Lys-Ala-Pro-Pro-Pro- |
| Ser-Leu-Pro-Ser-Pro-Ser-Arg-Leu-Pro-Gly-Pro- |
| Ser-Asp-Thr-Pro-Ile-Leu-Pro-Gln-Cys |

| |
|---|
| Structure (XIV) |
| Cys-Pro-Pro-Pro-Pro-Pro-Pro-Pro-Asp-Asp-Pro- |
| Arg-Phe-Gln-Asp-Ser-Ser-Ser-Ser-Lys-Ala-Pro- |
| Pro-Pro-Ser-Leu-Pro-Ser-Pro-Ser-Arg-Leu-Pro- |
| Gly-Pro-Ser-Asp-Thr-Pro-Ile-Leu-Pro-Gln |

Structure (XII) will be recognized as having the sequence of Structure (II) with the addition of Thr-Cys residues at its N-terminal. Structure (XIII) is similar to Structure (IX) but does not contain the spacer sequence. Structure (XIV) will be recognized as being similar to Structure (II) with the addition of spacer components at the N-terminal and a cysteine residue, which may be useful for modification reactions, as described in more detail below.

As already mentioned, it is only the 111-145 amino acid sequence of beta-HCG which differs from the corresponding sequence of LH. However, research indicates that the peptides used in the present conjugates may contain sequences corresponding to the 101-110 sequence which is common to beta-HCG and beta-LH without inducing the formation of antibodies reactive to LH. Thus, one can use in the instant conjugates peptides containing part or all of the common 101-110 sequence without causing substantial cross-reactivity with LH. For example, Structure (II) above represents the 111-145 amino acid sequence of beta-HCG. If desired, therefore, a peptide having the 101-145 amino acid of beta-HCG could be substituted for the peptide of Structure (II) in the instant conjugates without substantially affecting the activity of the modified polypeptide and without causing cross-reactivity with beta-LH. As already mentioned, a peptide corresponding to the 109-145 sequence of beta-HCG is especially useful in the present vaccines and conjugates.

For reasons already noted, the need to avoid cross-reactivity with luteinizing hormone mainly restricts the chorionic.gonadotropin-derived peptides used in the vaccines of the present invention to peptides containing all or part of the 101-145 sequence of chorionic gonadotropin, since it is only this part of the chorionic gonadotropin sequence which differs significantly from luteinizing hormone. However, it has been found that there are antigenic determinants on the human chorionic gonadotropin molecule that will produce human chorionic gonadotropin-specific antibodies, which antigenic determinants are not located on the 101-145 sequence of human chorionic gonadotropin. One such antigenic determinant is the sequence corresponding to the sequence 40-52, or the sequence 38-57, of the beta-subunit of human chorionic gonadotropin. Thus, peptides comprising an amino acid sequence substantially similar to the 38-57 region (or part of this region) of the beta-subunit of human chorionic gonadotropin can be used in the vaccines and conjugates of the present invention.

The beta-HCG(38-57) peptides are, however used in a manner rather different from the beta-HCG(101-145) peptides previously discussed. Since the 38-57 region of the beta-subunit of human chorionic gonadotropin is substantially similar to the corresponding region of human luteinizing hormone, follicle stimulating hormone and thyroid stimulating hormone (and the same is true in other species), it is not advisable to use the beta-HCG(38-57) peptides alone in the vaccines and conjugates of the invention, since this involves a substantial risk of producing antibodies with an undesirable degree of cross-reactivity with other hormones. However, as noted above, it is advantageous for the vaccines of the invention to comprise more than one antigenic determinant of the target protein, since this increases the efficacy of the vaccine or conjugate in raising antibodies, and thus produces a higher antibody titer in the treated animal. Accordingly, it is highly desirable that the beta-HCG(38-57) and analogous peptides be used in the vaccines in conjunction with a peptide which is more specific to human chorionic gonadotropin, in order that the vaccine or conjugate will be highly efficacious in raising antibodies, but will still be sufficiently specific that an undesirable level of cross-reaction is not experienced. In particular, it is recommended that the beta-HCG(38-57) peptide be used in conjunction with a peptide derived from, or similar to, the 109-145 sequence (or, for the reasons discussed above, the 101-145 sequence) of the same hormone subunit.

The joint use of the 38-57 and 101-145 peptides may be achieved in three separate, ways. Firstly, the betaHCG(38-57) peptide may further comprise one or more amino acid sequences substantially similar to at least part of the 101-145 region of the same hormone subunit; i.e., the two sequences may be chemically combined in the same peptide prior to modification of the peptide. Secondly, both peptides may be chemically linked to the same carrier without first being chemically bonded to one another. Finally, the two peptides may be bonded to separate carriers and a mixture of the two resultant conjugates introduced into the animal to be treated.

As used in the modified vaccines of the invention, the peptide comprising an amino acid sequence corresponding to the 38-57 region of the beta subunit of HCG is used in a form in which the two cysteine residues corresponding to the cysteine residues at positions 38 and 57 of the beta-subunit of HCG have their sulfur atoms linked in a disulfide bridge, since it appears to be only this form of the peptide, in which in effect the disulfide bridge close a loop, which has strongly antigenic properties in vivo. In the present state of chemical synthesis, it is in practice necessary to cyclize the 38-57 peptide before coupling it to a carrier (or to other peptide fragments) since the conditions necessary for cyclization cannot readily be produced after the peptide is coupled to a carrier (or to other peptide fragments).

Examples of specific preferred peptides having sequences analogous to the 38-57 region of HCG and useful in the vaccines of the present invention are as follows:

| (Structure XXV) |
|---|
| Cys-Pro-Ser-Met-Lys-Arg-Val-Leu-Pro-Val-Ile-Leu- |
| Pro-Pro-Met-Pro-Gln-Arg-Val-Cys |

| |
|---|
| (Structure XXVI) |
| Cys-Pro-Thr-Met-Met-Arg-Val-Leu-Gln-Ala-Val-Leu- |
| Pro-Pro-Leu-Pro-Gln-Val-Val-Cys; |

| (Structure XXVII) |
|---|
| Cys-Pro-Thr-Met-Thr-Arg-Val-Leu-Gln-Gly-Val-Leu- |
| Pro-Ala-Leu-Pro-Gin-Val-Val-Cys; |

| |
|---|
| (Structure XXVIII) |
| Cys-Tyr-Thr-Arg-Asp-Leu-Val-Tyr-Lys-Asn-Pro-Ala- |
| Arg-Pro-Lys-Ile-Gln-Lys-Thr-Cys; |

| |
|---|
| (Structure XXIX) |
| Cys-Tyr-Thr-Arg-Asp-Leu-Val-Tyr-Lys-Asp-Pro-Ala- |
| Arg-Pro-Lys-Ile-Gln-Lys- Thr-Cys; |

| |
|---|
| (Structure XXX) |
| Cys-Pro-Ser-Met-Val-Arg-Val-Thr-Pro-Ala-Ala-Leu- |
| Pro-Ala-Ile-Pro-Gln-Pro-Val-Cys; |

| |
|---|
| (Structure XXXI) |
| Cys-Met-lbr-Arg-Asp-lie-Asp-Gly-Lys-Leu-Phe-Leu- |
| Pro-(Lys-Tyr)-Ala-Leu-Ser-Gln-Asp-Val-Cys. |

Structure XXVII is the 38-57 region of human chorionic gonadotropin. Structure XXX is the corresponding sequence from equine chorionic gonadotropin. Structure XXVI is the corresponding region of human luteinizing hormone, and Structure XXV is the corresponding region of ovine/bovine luteinizing hormone. Structure XXVIII is the corresponding region of human follicle stimulating hormone, while Structure XXIX is the corresponding region of equine follicle stimulating hormone. Structure XXXI is the corresponding region of thyroid stimulating hormone. The (Lys-Tyr) portion of this hormone sequence is in parentheses because it represents an "insert" between positions 50 and 51 of the corresponding HCG sequence, and thus has no direct equivalent in any of the other sequences given above.

It should be noted that there are some differences of opinion among those skilled in the field of protein sequence determination as to certain minor details of the above sequences. See, for example:
Pierce and Parsons, Ann. Rev. Biochem. 50: 469-95 (1981).
In particular, some authorities dispute the existence of the aforementioned (Lys-Tyr) insert in the human thyroid stimulating hormone sequence, while other authorities dispute the existence of the methionine at position 42 and the valine at position 55 of the human luteinizing hormone sequence. However, for reasons discussed above, even if the natural sequences do differ from those just given, the sequences just given are certainly sufficiently close to the natural sequences to produce a strong antigenic reaction when incorporated into vaccines and conjugates of the invention.

### Relaxin

Another group of peptides which can be used in the present vaccines and conjugates are relaxin and polypeptides derived therefrom. It has been known for a long time that relaxin is a peptide hormone synthesized in the corpus luteum of ovaries during pregnancy and the hormone is released into the bloodstream prior to parturition. The major biological effect of relaxin is to remodel the mammalian reproductive tract to facilitate the birth process, primarily by relaxing the cervix, thereby assisting in the dilation of the cervix prior to parturition. The amino acid sequence; which bears some resemblance to that of insulin, has been determined; see:
Hudson et al, Structure of a Genomic Clone Encoding Biologically Active Human Relaxin.
This paper also gives methods for the synthesis of certain relaxin-derived peptides.

The use of relaxin or peptides derived therefrom depends not upon the natural function of relaxin during parturition, but upon the fact that anti-relaxin antibodies are known to render sperm immotile. Thus, there appears to be a relaxin-like antigen present on the surface of sperm, especially since the immotility of the sperm can be reversed by adding relaxin to the antibody/sperm complex. In theory one could use modified sperm antigens to generate in the male antibodies to various antigens present in sperm, but there is the serious problem that, owing to the blood/testes barrier, such anti-sperm antibodies do not penetrate the testes. The potentially very rapid induction of immotility of anti-relaxin antibody renders generation of such an antibody in males a highly attractive potential form of male contraception. Although the anti-relaxin antibodies will not penetrate the testes because of the blood/testes barrier, they can penetrate the epididymis and they will also be secreted into the fluid which becomes mixed with the sperm shortly before or during ejaculation. Thus, by producing anti-relaxin antibodies in the male, ejaculation would take place normally but the sperm produced would be immotile. Furthermore, the risk of complications and unintended tissue damage by such an instant process is slight, since the antibodies will not enter the testes, thereby avoiding potentially damaging reactions due to antibody-antigen binding within the testes.

It should be noted that injection of relaxin-derived conjugates of the present invention into females is not recommended; such a process would carry, too great a risk of ovarian damage in the female.

It should also be noted that relaxin is a highly species-specific protein. Accordingly, when choosing an appropriate peptide derived from relaxin, care should be taken to ensure that the peptide corresponds to part of the sequence of human relaxin (or, of course, relaxin of any other species which it is designed to treat).

### Cancer Treatment

Another health problem that can be treated is that of certain endocrine or hormone-dependent tumors or cancers. Certain breast cancers have been shown to be dependent upon the abundant secretion of the hormone prolactin for their continued survival. The inhibition of the secretion of prolactin has been shown to diminish the growth rate and the actual survival of certain of these tumors. The immunization of mammals suffering from such tumors with conjugates related to prolactin would result in the systematic reduction of the level of prolactin circulating in the system and consequently may result in the regression or remission of tumor growth. The consequence of this treatment would be far more favorable in terms of effective treatment of this disease, since surgical removal of the breast is the principal method of treatment currently available. It is of course likely that the vaccines of the present invention will be . effective only against those tumors which are dependent upon the secretion of prolactin (or some other hormone) for survival.

Investigators have also determined, for example, that certain polypeptide entities are supportive factors to, and secretions of, neoplastic diseases in both man and other mammals. These supportive entities have biochemically, biologically and immunologically close resemblances to hormones, particularly to CG as well as to 1.H. Using the vaccines of the instant invention the function of such polypeptides or endogenous counterparts can be neutralized to carry out regulation of the malignancy. For example, tumors in both male and female primates may be treated by isoimmunization procedures developing antibodies to CG or LH or the disease supportive factors analogous thereto. Furthermore, neoplasms in primate females may be regulated by isoimmunization procedures developing antibodies to endogenous LH. This hormone, when associated with a tumor state, tends to aggravate the tumorous condition.

It appears that certain carcinomas exude CG or an immunologically-similar material on their surfaces, thereby presenting to the immune system of the host animal a surface which, superficially, appears to be formed of material endogenous to the host animal and which is thus relatively non-immunogenic. Because of this known association between certain carcinomas and CG or CG-like materials, the CG-derived conjugates of the invention are useful for treatment of carcinomas associated with CG or CG-like materials. As already mentioned, vaccines of the invention are useful for the treatment of colon cancer

### Techniques for modification of Hormones. Fragments or Peptides

A wide range of techniques may be used to form the conjugates used in the present vaccines. Many of the techniques described below are not in themselves novel and some of the techniques may be found in the following list of literature references, while various others may be found elsewhere in literature by persons skilled in the art:
1. Klotz et al., Arch. of Biochem. and Biophys, 96,60 605-612, (1966).
2. Khorana, Chem. Rev. S3 145 (1953).
3. Sela et al., Biochem. J., 85, 223 (1962).
4. Eisen et al., J. Am. Chem. Soc., 75, 4583 (1953).
5. Centeno et al., Fed. Proc. (ABSTR), 25, 729 (1966).
6. Sokolowsky et al., J. Am. Chem. Soc., 86, 1212 (1964).
7. Tabachnick et al., J. Biol. Chem., 234, 1726, (1959).
8. Crampon et al., Proc. Soc. Exper. Biol. & Med, 80, 448 (1952).
9. Goodfriend et al., Science, 144, 1344 (1964).
10. Sela et al., J. Am. Chem. Soc., 78, 746 (1955).
11. Cinader et al., Brit. J. Exp. Pathol., 36, 515 (1955).
12. Phillips et al, J. of Biol. Chem., 240(2), 699-704 (1965).
13. Bahl, J. of Biol. Chem., 244, 575 (1969).

It will be appreciated by those skilled in the art that, in the instant invention, the chemical modification of the hormone, fragment or peptide is effected outside the body of the animal prior to introduction of the conjugate into the body of the animal.

Conjugation of hormones, fragments or peptides to chemically-modified diphtheria toxoid or T cell epitopes to form the vaccines and conjugates of the present invention may be effected by attaching to the hormone, fragment or peptide one or more foreign reactive (modifying) groups and/or by attaching two or more fragment peptides to a single foreign reactive group (i.e. a carrier) or both of the above, so that the body of the animal, recognizing the conjugate as a foreign object, produces antibodies which complex with not only the conjugate but also the unmodified hormone responsible for the disease or medical problem being regulated.

Particularly where the larger whole hormone or subunit type molecular structures are concerned, the number of foreign reactive groups which are to be attached to the hormone or subunit and the number of hormone or subunits to be attached to a foreign reactive group depends on the specific problem being treated. Basically, what is required is that the conjugate be modified to a degree sufficient to cause it to be antigenic when injected into the body of the animal. If too little modification is effected, the body may not recognize the conjugate as a foreign body and not create antibodies against it. If the number of foreign molecules added is too great, the body will create antibodies against the conjugate, but the antibodies will be specific to the conjugate and will not neutralize the action of the concerned natural endogenous hormone, i.e. they will be specific to the modifier.

In general, again considering the larger molecule subunit or whole hormone, it has been found that about 1-40 modifying groups per molecule of hormone or subunit will be useful in modifying the polypeptide adequately so as to obtain the desired immunological effect of this invention. As will be appreciated by one skilled in the art, this ratio of modifying groups will vary depending upon whether an entire hormone is utilized for modification or whether for instance a relatively small synthetic fragment of the hormone is to be modified. Generally for the larger molecules, it is preferred that 2-40 modifying groups per molecule of polypeptide be used according to this invention. In the instance where the polypeptide is the beta-subunit of HCG, it is particularly preferred that about 5-30 and more preferably 10-26 modifying groups per molecule of polypeptide be used. The degree of modification of the polypeptide should be adequate to induce generation, by the animal, of antibodies adequate to neutralize some of the target hormone or non-hormonal protein. The necessary degree of modification will vary with each polypeptide involved, and the degree of correction or change desired for the body function involved. As already noted, in the present vaccines, it is preferred that the conjugate comprise 20-30 peptides per 10⁵ daltons of the chemically-modified diphtheria toxoid.

It is preferred that the modification constitute two or more immunological determinants represented on the native protein to which it is desired to evoke an antibody response. The effect is one of heterogeneity of antibody development. Thus, several peptides have been described above having at least two distinct amino acid sequences represented in the HCG beta subunit. These sequences may be so spaced as to present the determinants in mutual isolation, while the spaced sequence fragment is conjugated with the carrier. Alternately, a mixed immunization arrangement may be utilized wherein a first peptide fragment developing one determinant is conjugated with a first carrier molecule and is administered in combination with a second, distinct peptide fragment which is conjugated with a second carrier molecule, the latter of which may be the same as or different in structure from the first carrier. Thus, each macromolecular carrier may be conjugated with hormone fragments such that each fragment represents two or more immunological determinants.

In one preferred modification process, the fragment to be modified, for example that designated Structure (XII) above, is activated first, after which it is conjugated with the carrier (chemically modified diphtheria toxoid or T cell epitope). An activating reagent may be utilized which exhibits differing functionality at its ends and, by choice of reaction conditions, these end functions can be made to react selectively. For example, the activators of Formulae A and B shown in Figure I of the accompanying drawings, which each have a maleiimido group and a substituted acid group, may be used. In these activators, X is a non-reacting group which can be a substituted or unsubstituted phenyl or C1-C10 alkylene moiety, or a combination thereof. The substituent on the phenyl ring (if any) should of course be non-interfering with the reactions of the activator, as should the remainder of the grouping X.

The grouping X may be, inter alia, a pentamethylene, 1,4-phenylene or monomethyl-1,4-phenylene grouping.

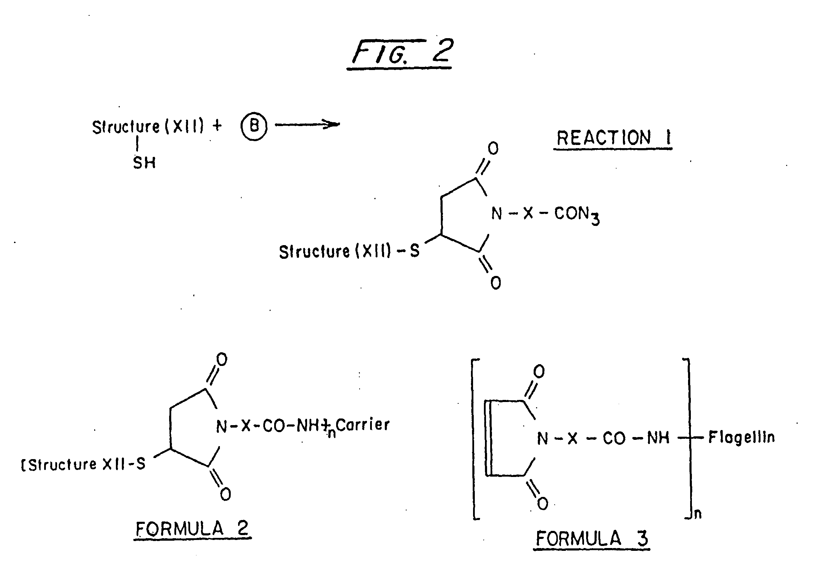

The maleiimido grouping of the above activators will react with sulfhydryl (SH) groups in the fragment to be modified under conditions whereby the opposite end (active ester end) of the reagent does not react with the amino groups present in the hormone, fragment or peptide. Thus, for example, peptides, such as that designated Structure (XII) above, contain a cysteine amino acid, and hence an SH group, react as shown in Reaction I in Figure 2 of the accompanying drawings. Following the above reaction, upon adjusting the pH to slightly alkaline condition, for example, pH δ, and adding the carrier, conjugation is accomplished to produce the product of Formula 2 show in Figure 2.

A carrier which does not contain SH groups, but does contain NH₂ groups, is preferably first treated with an activator of the formula A or B shown in Figure I, wherein X is as defined above, at pH 7 or lower to cause reaction of the active ester end of the activator with the carrier, giving a compound of Formula 3 shown in Figure 2. Thereafter, the activated carrier is reacted with a hormone, fragment or peptide containing a SH group to produce a conjugate similar to that discussed immediately above.

Should the fragment not contain an SH group, e.g. Structures (II), (III), (VI) and (VII), such structures can be modified first to introduce such a grouping by standard methods such as "thiolactonization", following which they are conjugated utilizing the above-discussed selective bi-functional reagents. For a more detailed description of these reagents, reference is made to the following publications:
O. Keller and J. Ridinger, Helv. Chim. Acta, 58, 531-541 (1975).
W. Trommer, H. Kolkenbrock and G. Pfleiderer, Hoppe-Seyler's Z. Physiol. Chem., 356, 1455-1453 (1975).

As already mentioned, in many natural proteins containing cysteine residues, these residues are not present in the thiol form containing a free SH group: instead, pairs of cysteine residues are linked by means of disulfide bridges to form cysteine. Accordingly, when it is desired to produce free SH groups in proteins to carry out the coupling reactions discussed above, one convenient way of providing such free SH groups may be to cleave disulfide bridges naturally present in the protein or other polypeptides which it is desired to conjugate. For example, as noted above the natural form of beta-HCG contains six disulfide bridges. To produce free thiol groups for coupling reactions, any number of these bridges from 1 to 6 may be broken using known techniques as set out for example in:
Bahl et al. Biochem. Biophys. Res. Comm., 70. 525-532 (1976).
This particular article describes cleavage of 3-5 of the six disulfide bridges in beta-HCG, but the same techniques may be used to break all six bridges if this is so desired. It should, however, be noted that the techniques disclosed in this paper are not selective and although it is possible to control the degree of disulfide bridge breaking, it is not possible to break specific bridges and leave others. The breaking of bridges is at random and the thiol groups produced are randomly distributed over the possible positions in beta-HCG.

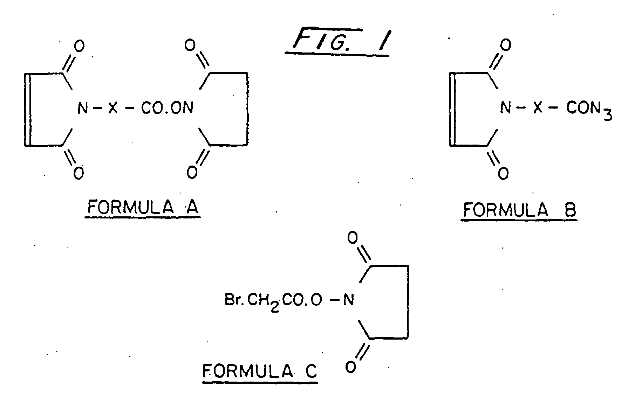

As an alternative approach to the utilization of the maleiimido group reagents discussed above, an alkylation step may be used to cause conjugation. Conditions can be chosen such that, in the presence of amino groups, essentially only thiol groups will be alkylated. With this approach, the larger carrier molecule is first modified by reaction of a fraction of its amino groups with an active ester of chloro, dichloro, bromo, or iodo acetic acid such as the compound of Formula C shown in Figure 1. This modified carrier is then reacted with the sulfhydryl group in the fragment to be modified (or a modified form of the polypeptide which has already been modified to contain a free thiol group (e.g. by the thiolactonization which is discussed above) if it did not originally posses such a free thiol group). The reaction produces a thioether linkage by alkylation of the free-thiol (sulfhydryl) group.

It may be seen from an observation of the formulae of Structures (IV). (V), (IX), (X), (XI), (XII), (XIII), and (XIV) that a Cys amino acid, which in a reduced state provides an SH reactive group, is located at either the C-terminal or N-terminal of the peptide structure. This location permits the peptide to be chemically linked to carrier molecules at either terminus. Moreover, the Structures (XIV), (X), (IX), (X), (IV) have a six-proline spacer chain (Pro)6 between the cysteine residue and the remainder of the peptide sequence. This latter arrangement provides a chemical spacer between the coupled carrier and the sequences representing a fragment of the natural hormone. A six-proline spacer can be added as a side chain spacer, for example at position 122 (lysine) in Structure (II). by initially adding an SH group (thiolactonization) to the free or unblocked epsilon amino group on this (lysine) residue. As previously noted, if such a spacer is employed, it desirably comprises from 2 to 8 amino acid residues. Then, utilizing the activator A or B in Figure 1 in which the component "X" is a chain of six proline amino acids, conjugation can be carried out. In the latter case, a spacer is provided between the carrier and peptide linked at an intermediate site, for example at position 122 in Structure (II). In the former case, only the spacer derived from the conjugating reagent links the carrier and peptide.

Carriers containing free amino groups can be prepared in buffer solution, such as phosphate buffer, in sodium chloride solution at a pH of 6-8. To this solution, tolylene diisocyanate (T.D.I.C.) reagent diluted from about 1-10 to about 1-40 times with dioxane can be added to the carrier. The general procedure was disclosed by Singer and Schick, J. Biophysical and Biochem. Cytology, 9, 519 (1961). The amount of T.D.I.C. added may range from 0.075 to 1,000 molar equivalents of the carrier used. The reaction may be carried out at about - 5° to about + 10°C, preferably 0° to 4° C, for about 1/2 to 2 hours. Any excess T.D.I.C. may be removed by centrifugation. The precipitate may be washed with the above-mentioned phosphate buffer and the supernatants combined.

This activated carrier solution may then be combined with the fragment to be conjugated. The fragment is dissolved in the same phosphate buffer (5-30 mg/ml) and the volumes of the two solution needed to provide the desired molar ratio of carrier to hormone, fragment or peptide in the conjugate are combined. The combined solutions are desirably reacted st 30°-50°C; preferably 35°-40°C, for 3-6-hours.

Separation of modified and unmodified fragment may be accomplished by conventional techniques, such as gel filtration.

Picogram amounts of I¹²⁵ labeled polypeptide may be added as a tracer to the reaction mixture at the time of conjugation, and the quantity of fragment conjugated to carrier (molar ratio) may be determined by the amount of radioactivity recovered.

Included in the methods for modifying the fragments are conjugation by use of water-soluble carbodiimide. The amino groups of the unmodified hormone, fragment or peptide are first preferably protected by acetylation. This (acetylated) unmodified, fragment is then conjugated to the carrier using 10-ethyl-3-(3-dimethylamino propyl)carbodiimide as activating agent. This method is generally disclosed by Hoare and Koshland, Jr., J. of Biological Chemistry, 242, 2447 (1967). The conjugation between the unmodified hormone, fragment or peptide and the carrier may be performed in a solvent such as glycine methyl ester while maintaining the pH at about 4-5, preferably about 4.5-4.8. The temperature of reaction is conveniently about room temperature and the reaction may be allowed to proceed for about 2-8 hours, preferably 5 hours. The resulting conjugate may be purified by conventional techniques, such as column chromatography.

Conjugates may also be prepared using glutaric dialdehyde as conjugating agent. According to a theory proposed by Richards and Knowles [J. Mol. Biol., 37, 231 (1968)], commercial glutaric dialdehyde contains virtually no free glutaric dialdehyde, but rather consists of a very complex mixture of polymers rich in alpha, beta-unsaturated aldehydes. Upon reaction with amino acid carriers, these polymers form a stable bond through the free amino group, leaving aldehyde groups free. This intermediate product then reacts with unmodified hormone, fragment or peptide in the presence of alkali metal borohydride, such as sodium borohydride. This intermediate is formed at pH 7-10, preferably 8-9, at about room temperature. The modified hormone, fragment or peptide is also conveniently obtained at about room temperature after about 1/4 -2 hours reaction time. The resulting product is recovered in pure form by conventional techniques, such as gel filtration, dialysis and lyophilization.

Throughout the foregoing description, the term "modified" or "conjugated" has been utilized in referring to the chemical reaction by which the carrier molecules become chemically attached to specific sites on the hormone, fragment or peptide. Although specific mechanisms by which this is accomplished are described herein in detail, other appropriate mechanisms may be used if desired. It is clear that the carrier can be a physically larger molecule or fragment thereof than the fragment which it modifies. Clearly, physical size of the carrier is not always critical (many of the T cell epitopes used in the conjugates of the present invention only contain about 15 to 20 amino acid residues), the criterion for effectiveness being that the mammalian body's reaction generate antibodies in sufficient quanta and specificity to the target hormone.

### Vaccines/Administration of the Conjugates

Obviously, in order that the conjugates can provoke the formation of antibodies to the target hormone within the body of an animal, they must be administered to the animal in such a way that they can come into contact with the cells responsible for formation of antibodies. In practice, this essentially means that the conjugates must be introduced into the circulatory system of the mammal to which they are administered. Although the use of other modes of administration is not absolutely excluded, in view of the molecular size and weight of most of the instant conjugates likely to be used in practice, the normal route or administration will be parental administration i.e. by injection. In the vast majority of cases, the quantity of conjugate which will need to be administered will be far too small for convenient handling alone, and in any case the chemical nature of most of the conjugates prevents them being produced in a pure form free from liquid vehicles. Accordingly, it is normally necessary to administer the conjugates as a vaccine comprising the conjugate together with a vehicle; this vaccine is desirably a vaccine of the present invention in which the conjugate and an adjuvant are dispersed in a phosphate-buffered saline aqueous vehicle, which is then emulsified with a mixture of oils, preferably a mixture of squalene and squalane oils, and mannide monooleate. It has been found that administration of the conjugate in such a vaccine has the effect of increasing the quantity of antibodies provoked by the conjugate when the vaccine is administered to an animal. To further increase the quantity of antibodies provoked by administration of the vaccine, it is advantageous to include in the vaccine an immunological adjuvant. The term "adjuvant" is used in its normal meaning to one skilled in the art of immunology, namely as meaning a substance which will elevate the total immune response of the animal to which the vaccine is administered i.e. the adjuvant is a non-specific immunostimulator. As already mentioned , the preferred adjuvant is N-acetyl-D-glucosamine-3-yl-acetyl-L-ala-D-isoglutamine (nor muramyl dipeptide), although other adjuvants, especially other muramyl dipeptides, for example:
NAc-nor Mur-L.Ala-D.isoGln
NAc-Mur-(6-0-stearoyl)-L.Ala-D.isoGln or
NGlycol-Mur-L.alphaAbu-D.isoGln
may be used if desired.

The vaccines of this invention may be administered parenterally to the animals to be protected, the usual modes of administration of the vaccine being intramuscular and sub-cutaneous injections. The quantity of vaccine to be employed will of course vary depending upon various factors, including the condition being treated and its severity. However, in general, unit doses of 0.1-50 mg. in large mammals administered from. one to five times at intervals of 1 to 5 weeks provide satisfactory results. Primary immunization may also be followed by "booster" immunization at 1 to 12 month intervals.

Other useful administration methods for the conjugates include those wherein the conjugate itself, or a solution or an emulsion thereof, is encased in a pharmaceutically acceptable polymer composition, such as in microcapsule form. The microencapsulated conjugate is then administered, for example, by implantation under the skin or intramuscular injection, so as to permit a controlled and/or prolonged and/or timed release of the conjugate. This release of the conjugate in turn elicits a controlled, prolonged, timed or as desired, raising of useful antibodies for purposes described herein. Illustrative of useful polymer compositions for the encapsulation include pharmaceutically acceptable polylactic-polyglycolic acid copolymers known in the art for pharmaceutical microencapsulation.

The following Examples are now given, though by way of illustration only, to show details of particularly preferred reagents, conditions and techniques used in the present invention.

### Example 1

### Preparation of peptide

Beta-HCG(109-145) was synthesized by solid phase synthesis, as described in the aforementioned U.S. Patents Nos. 4,855,285 and 5,006,334. It should be noted that only a peptide containing a free thiol group can be conjugated to the toxoid, and accordingly it is advisable to determine the thiol content of the peptide quantitatively by Elman's method before conjugating. If necessary, either N-acetyl homocysteine thiolactone or N-succinimidyl-3-(2-pyridylthio)propionate may be used to provide free thiol groups on the peptide.

### Preparation of modified diphtheria toxoid

Diphtheria toxoid, of the type used commercially to prepare diphtheria-pertussis-tetanus vaccines, was obtained from a commercial source. The toxoid was obtained in liquid form having an activity of 1000-1500 Lf/ml, and containing 1/5000 thimerosal as preservative. To this toxoid, ethylenediamine (0.1 M) was added at a ratio of 2 mL per 100 mL of the liquid toxoid, and the resultant mixture was concentrated approximately ten-fold by ultrafiltration. The concentrate was gel filtered using either Sephadex G-50 or Biogel P-60 in 0.2 M ammonium bicarbonate. The non-retarded volume was collected and lyophilized.

To determine the amino content of the modified toxoid, the fluorescamine method was used. A small volume of sample solution containing approximately 1.0 mg/mL of modified toxoid was prepared, and a 20 µL aliquot assayed against a standard curve of α-N-acetyl-L-lysine samples containing from 10⁻⁹ to 10⁻⁸ moles.

### Coupling of peptide to toxoid

Since the maleimido group is light sensitive, the steps of the following preparation using this group are desirably conducted in darkness or subdued lighting.

To prepare an antigenic conjugate comprising approximately 25 peptides per 10⁵ daltons of toxoid, 20 mg of the chemically-modified toxoid prepared above was dissolved in 1 mL of 0.5 M aqueous sodium phosphate buffer, pH 6.6. Separately, 6.18 mg of 6-maleimido caproic acyl succinyl ester (MCS) was dissolved in 100 µL of purified, dried dimethylformamide (DMF). 25 µL of the resultant DMF solution was added over a period of 15 minutes to the stirred toxoid solution at room temperature. After the addition was complete, the resultant solution was stirred for a further 75 minutes, and the excess MCS and reaction by-products were removed by gel filtration on Sephadex G-25 equilibrated with 0.1 M sodium citrate/0.1 M EDTA, pH 6.0, at 0-4°C. The activated toxoid excluded from the column was concentrated to approximately 20 mg/mL by ultrafiltration on P10 membrane.

To effect the conjugation of the peptide to the modified toxoid, the solution of the modified toxoid prepared above was added to a solution of the peptide in the same sodium citrate/EDTA, pH 6.0 buffer, and the resultant mixture is stirred under a stream of nitrogen, while sealed from light, for 18 hours at room temperature. The solution was then passed through a Sephadex G-50 or Bio Gel P60 column equilibrated with 0.2 M ammonium bicarbonate at 0-4°C, and the conjugate eluted in the void volume was lyophilized.

### Preparation of vaccine

2.0 mg of the conjugate thus prepared and 1.0 mg of nor muramyl dipeptide were dissolved in 0.6 mL of phosphate-buffered saline solution. Separately 0.6 mL of an oil mixture was prepared comprising 44 parts by weight of squalene, 41 parts by weight of squalane, 11 parts by weight of mannide monooleate and 4 parts by weight of aluminum monostearate. Emulsification was effected by hand using an apparatus comprising two glass syringes connected via a three-way stopcock. The buffer solution was placed in one syringe, the oil mixture in the other, and after mixing the resultant mixture was passed back and forth at least 25 times until the resultant emulsion became quite viscous.

The vaccine thus produced was suitable for intramuscular injection in doses of 0.5-1.5 mL in humans.

### Example 2

This Example illustrates the preparation of preferred antigenic conjugates of the present invention containing T-cell epitopes.

These conjugates were synthesized using-the same solid state synthesis technique mentioned in Example 1 above. The T-cell peptide was synthesized first on the resin, then a spacer sequence of 4-5 amino acid residues containing a β-turn was added. Finally, the beta-HCG(109-145) peptide was synthesized on the N-terminal of the spacer sequence.

In a second method, a peptide was prepared having a terminal lysine. The primary amino group of the lysine was blocked, leaving the ε-amino group free. A T-cell or beta-HCG(109-145) peptide was synthesized as a side chain on the lysine ε-amino group, and back sequenced to assure the correct sequence. The blocking group on the lysine was removed and the main chain extended, with the last amino acid residue added being another lysine. The side chain synthesis was then repeated: Further repetitions of this procedure yielded a peptide containing two different T-cell epitopes and two beta-HCG(109-145) peptides.

Also, in a preferred process of this invention, on a resin using an Fmoc/ t-butyl technique was formed a β-strand template having the sequence:

Gly-Leu-Lys-Leu-Lys-Leu-Gly-COOH,

with both lysines having their ε-amino groups protected with Boc groups. The N-terminus of this template peptide was protected by the addition of Nps-Leu, and then the ε-blocking group was removed from the lysine adjacent the N-terminus of the peptide. Thereafter, a T-cell epitope was assembled on the free ε-amino group of the lysine using the Boc/benzyl technique described in aforementioned U.S. patents. The N-terminus of the T-cell epitope was blocked by acetylation, and then the Npys protecting group was removed from the N-terminus of the template. The main chain of the peptide was extended using the Fmoc/t-butyl technique to produce the sequence:

(Ac)Gly-Leu-Lys-Leu-Lys-Leu-Leu-Gly-Gly-Ser-Pro-Leu-Gly-Leu-Lys-Leu-Lys-Leu-Gly-COOH,

in which, as indicated by "(Ac)" the N-terminus was protected by acetylation. (The Gly-Ser-Pro-Leu sequence provides a β-turn spacer in the template.) The ε-blocking group was removed from the lysine adjacent the N-terminus of the peptide, and a B-cell epitope was assembled on the free ε-amino group of the lysine using the Fmoc/t-butyl technique. The N-terminal of the B-cell epitope was blocked by acylation. Finally, the t-butyl and Boc protecting groups were removed with TFA, and then the peptide was cleaved from the resin by low/high HF treatment.
Conjugates containing the following T-cell epitopes were prepared by these two procedures:
a. amino acids 580-599 of tetanus toxoid;
b. amino acids 830-844 of tetanus toxoid;
c. amino acids 916-932 of tetanus toxoid;
d. amino acids 947-967 of tetanus toxoid;
e. amino acids. 288-302 of measles virus protein;
f. amino acids 16-33 of hepatitis B viral protein; or
g. amino acids 317-336 of malaria CSP protein.

## Claims

1. A vaccines for treating colon cancer comprising:
a) an antigenic polypeptide fragment consisting of amino acids 101 to 145 of the beta subunit of human chorionic gonadotropin, amino acids 109 to 145 of the beta subunit of human chorionic gonadotropin or selected from the group consisting of structures II, III, IV, V, VI, VII, VIII, VIIIa, IX, X, XI, XII, XIII and XIV
| Structure (II) |
|---|
| Asp-Asp-Pro-Arg-Phe-Gln-Asp-Ser-Ser-Ser-Ser- |
| Lys-Ala-Pro-Pro-Pro-Ser-Leu-Pro-Ser-Pro-Ser- |
| Arg-Leu-Pro-Gly-Pro-Ser-Asp-Thr-Pro-Ile-Leu- |
| Pro-Gln |
| |
|---|
| Structure (III) |
| Gln-Asp-Ser-Ser-Ser-Ser-Lys-Ala-Pro-Pro-Pro- |
| Ser-Leu-Pro-Ser-Pm-Ser-Arg-Leu-Giy-Pro-Ser- |
| Asp-Thr-Pro-Ile-Leu-Pro-Gln |
| |
|---|
| Structure (IV) |
| Cys-Pro-Pro-Pro-Pro-Pro-Pro-Ser-Asp-Thr-Pro- |
| Ile-Leu-Pro-Gln |
| |
|---|
| Structure (V) |
| Asp-Asp-Pro-Arg-Phe-Gln-Asp-Ser-Pro-Pro-Pro- |
| Pro-Pro-Pro-Cys |
| |
|---|
| Structure (VI) |
| Phe-Gln-Asp-Ser-Ser-Ser-Ser-Lys-Ala-Pro-Pro- |
| Pro-Ser-Leu-Pro-Ser-Pro-Ser-Arg-Leu-Pro-Gly- |
| Pro-Ser-Asp-Thr-Pro-Ile-Leu-Pro-Gln |
| |
|---|
| Structure (VII) |
| Asp-Asp-Pro-Arg-Phe-Gln-Asp-Ser-Ser-Ser-Ser- |
| Lys-Ala-Pro-Pro-Pro-Ser-Leu-Pro-Ser |
| |
|---|
| Structure (VIII) |
| Asp-Asp-Pro-Arg-Phe-Gln-Asp-Ser-Pro-Pro-Pro- |
| Cys-Pro-Pro-Pro-Ser-Asp-Thr-Pro-Ile-Leu-Pro-Gln |
| |
|---|
| Structure (VIIIa) |
| Asp-Asp-Pro-Arg-Phe-Gln-Asp-Ser-Pro-Pro-Pro- |
| Pro-Pro-Pro-Cys-Pro-Pro-Pro-Pro-Pro-Pro-Ser- |
| Asp-Thr-Pro-Ile-Leu-Pro-Gln |
| Structure (EX) |
|---|
| Asp-His-Pro-Leu-Thr-Ala-Asp-Asp-Pro-Arg-Phe- |
| Gln-Asp-Ser-Ser-Ser-Ser-Lys-Ala-Pro-Pro-Pro- |
| Ser-Leu-Pro-Ses-Pro-Ser-Arg-Leu-Pro-Gly-Pro- |
| Ser-Asp-Thr-Pro-Ile-Leu-Pro-Gln-Pro-Pro-Pro- |
| Pro-Pro-Pro-Cys |
| |
|---|
| Structure (X) |
| Asp-Asp-Pro-Arg-Phe-Gln-Asp-Ser-Ser-Ser-Ser- |
| Lys-Ala-Pro-Pro-Pro-Ser-Leu-Pro-Ser-Pro-Ser- |
| Arg-Leu-Pro-Gly-Pro-Ser-Asp-Thr-Pro-Ile-Leu- |
| Pro-Gln-Pro-Pro-Pro-Pro-Pro-Pro-Cys |
| |
|---|
| Structure (XI) |
| Asp-Asp-Pro-Arg-Phe-Gln-Asp-Ser-Ser-Ser-Ser- |
| Lys-Als-Pro-Pro-Pro-Ser-Leu-Pro-Ser-Pro-Ser- |
| Arg-Leu-Pro-Gly-Pro-Ser-Asp-Thr-Pro-Ile-Leu- |
| Pro-Gln-Cys |
| |
|---|
| Structure (XII) |
| Thr-Cys-Asp-Asp-Pro-Arg-Phe-Gln-Asp-Ser-Ser- |
| Ser-Ser-Lys-Ala-Pro-Pro-Pro-Ser-Leu-Pro-Ser- |
| Pro-Ser-Arg-Leu-Pro-Cly-Pro-Ser-Asp-Thr-Pro- |
| De-Leu-Pro-Gln |
| |
|---|
| Structure (XIII) |
| Asp-His-Pro-Leu-Thr-Aba-Asp-Asp-Pro-Arg-Phe- |
| Gln-Asp-Ser-Ser-Ser-Ser-Lys-Ala-Pro-Pro-Pro- |
| Ser-Leu-Pro-Ser-Pro-Ser-Arg-Leu-Pro-Gly-Pro- |
| Ser-Asp-Thr-Pro-Ile-Leu-Pro-Gln-Cys |
| |
|---|
| Structure (XIV) |
| Cys-Pro-Pro-Pro-Pro-Pro-Pro-Pro-Asp-Asp-Pro- |
| Arg-Phe-Gln-Asp-Ser-Ser-Ser-Ser-Lys-Ala-Pro- |
| Pro-Pro-Ser-Leu-Pro-Ser-Pro-Ser-Arg-Leu-Pro- |
| Gly-Pro-Ser-Asp-Thr-Pro-Ile-Leu-Pro-Gln |
b) an antigenic polypeptide fragment analogous to amino acids 38-57 of the beta subunit of human chorionic gonadotropin selected from the group consisting of structures XXV, XXVI, XXVII, XXVIII, XXIX, XXX and XXXI
| (Structure XXV) |
|---|
| Cys-Pro-Ser-Met-Lys-Arg-Val-Leu-Pro-Val-Ile-Leu- |
| Pro-Pro-Met-Pro-Gln-Arg-Val-Cys; |
| |
|---|
| (Structure XXVI) |
| Cys-Pro-Thr-Met-Met-Arg-Val-Leu-Gln-Ala-Val-Leu- |
| Pro-Pro-Leu-Pro-Gln-Val-Val-Cys; |
| |
|---|
| (Structure XXVII) |
| Cys-Pro-Thr-Met-Thr-Arg-Val-Leu-Gln-Gly-Val-Leu- |
| Pro-Ala-Leu-Pro-Gln-Val-Val-Cys; |
| |
|---|
| (Structure XXVIII) |
| Cys-Tyr-Thr-Arg-Asp-Leu-Val-Tyr-Lys-Asn-Pro-Ala- |
| Arg-Pro-Lys-Ile-Gln-Lys-Thr-Cys; |
| |
|---|
| (Structure XXIX) |
| Cys-Tyr-Thr-Arg-Asp-Leu-Val-Tyr-Lys-Asp-Pro-Ala- |
| Arg-Pro-Lys-De-Gln-Lys-Thr-Cys; |
| |
|---|
| (Structure XXX) |
| Cys-Pro-Ser-Met-Val-Arg-Val-Thr-Pro-Ala-Ala-Leu- |
| Pro-Ala-Ile-Pro-Gln-Pro-Val-Cys; |
| |
|---|
| (Structure XXXI) |
| Cys-Met-Thr-Arg-Asp-Ile-Asp-Gly-Lys-Leu-Phe-Leu- |
| Pro-(Lys-Tyr)-Ala-Leu-Ser-Gln-Asp-Val-Cys. |
,wherein the two cystein residues are linked to form a disulfide bridge,
said antigenic polypeptide fragments a) and b) modified by conjugation at the N-terminus or C-terminus with a carrier consisting of a spacer peptide containing from about 2-8 amino acids and either a diphteria toxoid or a peptide fragment selected from the group of epitope sequences consisting of
a) amino acids 580-599 of tetanus toxoid
b) amino acids 830-844 of tetanus toxoid
c) amino acids 916-932 of tetanus toxoid
d) amino acids 947-967 of tetanus toxoid
e) amino acids 288-302 of measles virus protein
f) amino acids 16-33 of heparitis B vital protein
g) amino acids 317-336 of malaria CSP protein and
h) mixtures of said epitopes a) to g), and
squalane and/or squalene as an emulsifying agent and an adjuvant,
wherein said antigenic polypeptides are combined to form a fusion peptide, wherein said antigenic polypeptides a) and b) are separately conjugated to the carrier, or wherein said antigenic polypeptides a) and b) are conjugated to different carriers and are used as a mixture.

2. The vaccines according to claims 1 wherein the diphtheria toxoid is a reaction product of diphtheria toxoid with ethylenediamine.

3. The vaccines according to claims 1 and 2, wherein the antigenic conjugate comprises 20-30 polypeptides per 10⁵ daltons of the modified diphtheria toxoid.

4. The vaccines according to anyone of claims 1-3, containing additionally mannide monooleate and aluminium monostearate.

5. The vaccine according to claim 4, comprising by weight from 35 to 45 percent of squalene, from 35 to 45 percent of squalane, from 6 to 16 percent of mannide monooleate and from 1 to 5 percent of aluminium monostearate.

6. The vaccine according to anyone of claims 1-5-, wherein the adjuvant is N-acetyl-D-glucosamine-3-yl-acetyl-L-ala-D-isoglutamine (nor muramyl dipeptide).

7. The vaccine according to anyone of claims 1-6, comprising 0.2 to 1.0 mg of the adjuvant per ml vaccine.

8. The vaccine according to anyone af claims 1-7, wherein the content of the antigenic conjugate is 0.5 to 2.0 mg per ml vaccine.

9. The vaccine according to anyone of claims 1-8, wherein the vaccine comprises equal amounts of the aqueous phase and the emulsifying agent.

10. The vaccine according to anyone of claims 1-9, wherein the aqueous phase is phosphate-buffered saline.

11. The use of the vaccine according to anyone of claims 1-10 for the preparation of a medicament for the treatment of colon cancer

## Patentansprüche

1. Ein Impfstoff zur Behandlung von Darmkrebs umfassend:
a) ein antigenes Polypeptidfragment bestehend aus Aminosäuren 101 bis 145 der Beta-Untereinheit des humanen Choriongonadotropin, Aminosäuren 109 bis 145 des humanen Choriongonadotropin oder ausgewählt au der Gruppe bestehend aus den Strukturen II, III, IV, V, VI, VII, VIII, VIIIa, IX, X, XI, XII, XIII und XIV
| Struktur (II) |
|---|
| Asp-Asp-Pro-Arg-Phe-Gln-Asp-Ser-Ser-Ser-Ser- |
| Lys-Ala-Pro-Pro-Pro-Ser-Leu-Pro-Ser-Pro-Ser- |
| Arg-Leu-Pro-Gly-Pro-Ser-Asp-Thr-Pro-Ile-Leu- |
| Pro-Gln |
| |
|---|
| Struktur (III) |
| Gln-Asp-Ser-Ser-Ser-Ser-Lys-Ala-Pro-Pro-Pro- |
| Ser-Leu-Pro-Ser-Pro-Ser-Arg-Leu-Gly-Pro-Ser- |
| Asp-Thr-Pro-Ile-Leu-Pro-Gln |
| |
|---|
| Struktur (IV) |
| Cys-Pro-Pro-Pro-Pro-Pro-Pro-Ser-Asp- Thr-Pto- |
| Ile-Leu-Pro-Gln |
| |
|---|
| Struktur (V) |
| Asp-Asp-Pro-Arg-Phe-Gln-Asp-Ser-Pro-Pro-Pro- |
| Pro-Pro-Pro-Cys |
| |
|---|
| Struktur (VI) |
| Phe-Gln-Asp-Ser-Ser-Ser-Ser-Lys-Ala-Pro-Pro- |
| Pro-Ser-Leu-Pro-Ser-Pro-Ser-Arg-Leu-Pro-Gly- |
| Pro-Ser-Asp-Thr-Pro-Ile-Leu-Pro-Gln |
| |
|---|
| Struktur (VII) |
| Asp-Asp-Pro-Arg-Phe-Gln-Asp-Ser-Ser-Ser-Ser- |
| Lys-Ala-Pro-Pro-Pro-Ser-Leu-Pm-Ser |
| |
|---|
| Struktur (VIII) |
| Asp-Asp-Pro-Arg-Phe-Gln-Asp-Ser-Pro-Pro-Pro- |
| Cys-Pro-Pro-Pro-Ser-Asp-Thr-Pro-Ile-Leu-Pro-Gln. |
| |
|---|
| Struktur (VIIIa) |
| Asp-Asp-Pro-Arg-Phe-Gln-Asp-Ser-Pro-Pro-Pro- |
| Pro-Pro-Pro-Cys-Pro-Pro-Pro-Pro-Pro-Pro-Ser- |
| Asp-Thr-Pro-Ile-Leu-Pro-Gln |
| Struktur (IX) |
|---|
| Asp-His-Pro-Leu-Thr-Ala-Asp-Asp-Pro-Arg-Phe- |
| Gln-Asp-Ser-Ser-Ser-Ser-Lys-Ala-Pro-Pro-Pro- |
| Ser-Leu-Pro-Ser-Pro-Ser-Arg-Leu-Pro-Gly-Pro- |
| Ser-Asp-Thr-Pro-Ile-Leu-Pro-Gln-Pro-Pro-Pro- |
| Pro-Pro-Pro-Cys |
| |
|---|
| Struktur (X) |
| Asp-Asp-Pro-Arg-Phe-Gln-Asp-Ser-Ser-Ser-Ser- |
| Lys-Ala-Pro-Pro-Pro-Ser-Leu-Pro-Ser-Pro-Ser- |
| Arg-Leu-Pro-Gly-Pro-Ser-Asp-Thr-Pro-Ile-Leu- |
| Pro-Gln-Pro-Pro-Pro-Pro-Pro-Pro-Cys |
| |
|---|
| Struktur (XI) |
| Asp-Asp-Pro-Arg-Phe-Gln-Asp-Ser-Ser-Ser-Ser- |
| Lys-Ala-Pro-Pro-Pro-Ser-Leu-Pro-Ser-Pro-Ser- |
| Arg-Leu-Pro-Gly-Pro-Ser-Asp-Thr-Pro-Ile-Leu- |
| Pro-Gln-Cys |
| |
|---|
| Struktur (XII) |
| Thr-Cys-Asp-Asp-Pro-Arg-Phe-Gln-Asp-Ser-Ser- |
| Ser-Ser-Lys-Ala-Pro-Pro-Pro-Ser-Leu-Pro-Ser- |
| Pro-Ser-Arg-Leu-Pro-Gly-Pro-Ser-Asp-Thr-Pro- |
| Ile-Leu-Pro-Gln |
| |
|---|
| Struktur (XIII) |
| Asp-His-Pro-Leu-Thr-Aba-Asp-Asp-Pro-Arg-Phe- |
| Gln-Asp-Ser-Ser-Ser-Ser-Lys-Aln-Pro-Pro-Pro- |
| Ser-Leu-Pro-Ser-Pro-Ser-Arg-Leu-Pro-Gly-Pro- |
| Ser-Asp-Thr-Pro-Ile-Leu-Pro-Gln-Cys |
| |
|---|
| Struktur (XIV) |
| Cys-Pro-Pro-Pro-Pro-Pro-Pro-Pro-Asp-Asp-Pro- |
| Arg-Phe-Gln-Asp-Ser-Ser-Ser-Ser-Lys-Ala-Pro- |
| Pro-Pro-Ser-Leu-Pro-Ser-Pro-Ser-Arg-Leu-Pro- |
| Gly-Pro-Ser-Asp-Thr-Pro-Ile-Leu-Pro-Gln |
b) einem antigenen Polypeptidfragment, welches analog ist zu den Aminosäuren 38-57 der Beta-Untereinheit des Choriongonadotropin ausgewählt aus der Gruppe bestehend aus Strukturen XXV, XXVI, XXVII, XXVIII, XXIX, XXX und XXXI
| Struktur (XXV) |
|---|
| Cys-Pro-Ser-Met-Lys-Arg-Val-Leu-Pro-Val-Ile-Leu- |
| Pro-Pro-Met-Pro-Gln-Arg-Val-Cys; |
| |
|---|
| Struktur (XXVI) |
| Cys-Pro-Thr-Met-Met-Arg-Val-Leu-Gln-Ala-Val-Leu- |
| Pro-Pro-Leu-Pro-Gln-Val-Val-Cys; |
| |
|---|
| Struktur (XXVII) |
| Cys-Pro-Thr-Met-Thr-Arg-Val-Leu-Gln-Gly-Val-Leu- |
| Pro-Ala-Leu-Pro-Gln-Val-Val-Cys; |
| |
|---|
| Struktur (XXVIII) |
| Cys-Tyr-Thr-Arg-Asp-Leu-Val-Tyr-Lys-Asn-Pro-Ala- |
| Arg-Pro-Lys-Ile-Gln-Lys-Thr-Cys; |
| |
|---|
| Struktur (XXIX) |
| Cys-Tyr-Thr-Arg-Asp-Leu-Val-Tyr-Lys-Asp-Pro-Ala- |
| Arg-Pro-Lys-Ile-Gln-Lys-Thr-Cys; |
| |
|---|
| Struktur (XXX) |
| Cys-Pro-Ser-Met-Val-Arg-Val-Thr-Pro-Ala-Ala-Leu- |
| Pro-Als-Ile-Pro-Gln-Pro-Val-Cys ; |
| |
|---|
| Struktur (XXXI) |
| Cys-Met-Thr-Arg-Asp-Ile-Asp-Gly-Lys-Leu-Phe-Leu- |
| Pro-(Lys-Tyr)-Ala-Leu-Ser-Gln-Asp-Val-Cys. |
wobei die zwei Cyteinreste verbunden sind, um eine Disulfidbrücke zu bilden,
wobei die antigenen Polypeptidfragmente a) und b) durch Konjugation am N-Terminus oder am C-Terminus mit einem Träger modifiziert sind, der aus einem Spacer-Peptid besteht, das ungefähr 2-8 Aminosäuren beinhaltet und entweder ein Diphteria-Toxoid oder ein Peptidfragment ist ausgewählt aus der Gruppe von Epitopsequenzen bestehend aus:
a) Aminosäuren 580-599 des Tetanus-Toxoids
b) Aminosäuren 830-844 des Tetanus-Toxoiss
c) Aminosäuren 916-932 des Tetanus-Toxoids
d) Aminosäuren 947-967 des Tetanus-Toxoids
e) Aminosäuren 288-302 des Masernvirusproteins
f) Aminosäuren 16-33 des Hepatitis-B-Virusproteins
g) Aminosäuren 317-336 des Malaria-CSP-Proteins und
h) Mischungen besagter Epitope a) bis g) und
Squalan und/oder Squalen als Emulgator oder Adjuvans,
wobei die antigenen Popypeptide kombiniert sind, um ein Fusionspeptid zu bilden,
wobei die antigenen Polypeptide a) bis b) separat an den Träger konjugiert sind, oder
wobei die antigenen Polypeptide a) und b) an verschiedene Träger konjugiert sind und als Mischung verwendet werden.

2. Die Impfstoffe gemäß Anspruch 1, wobei das Diphteria-Toxoid ein Reaktionsprodukt des Diphteria-Toxoids mit Ethylendiamin ist.

3. Die Impfstoffe gemäß der Ansprüche 1 und 2, wobei das antigene Konjugat 20-30 Polypeptide pro 10⁵ Daltons des modifizierten Diphteria-Toxoids umfasst.

4. Die Impfstoffe gemäß jedem der Ansprüche 1-3, die zusätzlich Mannidmonooleat und Aluminiummonostearat enthalten.

5. Der Impfstoff gemäß Anspruch 4, umfassend von 35 bis 45 Gewichtsprozent Squalen, von 35 bis 45 Gewichtsprozent Squalan, von 6 bis 16 Gewichtsprozent Mannidmonooleat und von 1bis 5 Gewichtsprozent Aluminiummonostearat.

6. Der Impfstoff gemäß jedem der Ansprüche 1-5, wobei das Adjuvans N-Acetyl-D-glucosamin-3-yl-acetyl-L-ala-D-isoglutamin (Normuramyldipeptid) ist.

7. Der Impfstoff gemäß jedem der Ansprüche 1-6, umfassend 0.2 bis 1.0 mg des Adjuvans pro ml Impfstoff.

8. Der Impfstoff gemäß jedem der Ansprüche 1-7, wobei der Gehalt an antigenem Konjugat 0.5 bis 2.0 mg pro ml Impfstoff beträgt.

9. Der Impfstoff gemäß jedem der Ansprüche 1-8, wobei der Impfstoff gleiche Mengen an wässriger Phase und Emulgator umfasst.

10. Der Impfstoff gemäß jedem der Ansprüche 1-9, wobei die wässrige Phase phosphatgepufferte Kochsalzlösung ist.

11. Die Verwendung des Impfstoffes gemäß einem der Ansprüche 1-10 zur Herstellung eines Medikaments zur Behandlung von Darmkrebs.

## Revendications

1. Vaccin destiné au traitement du cancer du côlon, qui comprend:
a) un fragment de polypeptide antigénique constitué par les acides aminés 101 à 145 de la sous-unité bêta de la gonadotropine chorionique humaine, les acides aminés 109 à 145 de la sous-unité bêta de la gonadotropine chorionique humaine, ou choisi dans le groupe constitué par les structures II, III, IV, V, VI, VII, VIII, VIIIa, IX, X, XI, XII, XIII et XIV
| Structure (II) |
|---|
| Asp-Asp-Pro-Arg-Phe-Gln-Asp-Ser-Ser-Ser-Ser- |
| Lys-Ala-Pro-Pro-Pro-Ser-Leu-Pro-Ser-Pro-Ser- . |
| Arg-Leu-Pro-Gly-Pro-Ser-Asp-Thr-Pro-Ile-Leu- |
| Pro-Gln |
| |
|---|
| Structure (III) |
| Gln-Asp-Ser-Ser-Ser-Ser-Lys-Ala-Pro-Pro-Pro- |
| Ser-Leu-Pro-Ser-Pro-Ser-Arg-Leu-Gly-Pro-Ser- |
| Asp- Thr-Pro-Ile-Leu-Pro-Gln |
| |
|---|
| Structure (IV) |
| Cys-Pro-Pro-Pro-Pro-Pro-Pro-Ser-Asp-Thr-Pro- |
| Ile-Leu-Pro-Gln |
| |
|---|
| Structure (V) |
| Asp-Asp-Pro-Arg-Phe-Gln-Asp-Ser-Pro-Pro-Pro- |
| Pro-Pro-Pro-Cys |
| |
|---|
| Structure (VI) |
| Phe-Gln-Asp-Ser-Ser-Ser-Ser-Lys-Ala-Pro-Pro- |
| Pro-Ser-Leu-Pro-Ser-Pro-Ser-Arg-Leu-Pro-Gly- |
| Pro-Ser-Asp-Thr-Pro-Ile-Leu-Pro-Gln |
| |
|---|
| Structure (VII) |
| Asp-Asp-Pro-Arg-Phe-Gln-Asp-Ser-Ser-Ser-Ser- |
| Lys-Ala-Pro-Pro-Pro-Ser-Leu-Pro-Ser |
| |
|---|
| Structure (VIII) |
| Asp-Asp-Pro-Arg-Phe-Gln-Ser-Pro-Pro-Pro- |
| Cys-Pro-Pro-Pro-Ser-Asp-Thr-Pro-Ile-Leu-Pro-Gln |
| |
|---|
| Structure (VIIIa) |
| Asp-Asp-Pro-Arg-Phe-Gln-Asp-Ser-Pro-Pro-Pro- |
| Pro-Pro-Pro-Cys-Pro-Pro-Pro-Pro-Pro-Pro-Ser- |
| Asp-Thr-Pro-Ile-Leu-Pro-Gln |
| Structure (IX) |
|---|
| Asp-His-Pro-Leu-Thr-Ala-Asp-Asp-Pro-Arg-Phe- |
| Gln-Asp-Ser-Ser-Ser-Sef-Lys-Ala-Pro-Pro-Pro- |
| Ser-Leu-Pro-Set-Pro-Ser-Arg-Leu-Pro-Gly-Pro- |
| Ser-Asp-Thr-Pro-Ile-Leu-Pro-Gln-Leu-Pro-Pro-Pro- |
| Pro-Pro-Pro-Cys |
| |
|---|
| Structure (X) |
| Asp-Asp-Pro-Arg-Phe-Gln-Asp-Ser-Ser-Ser-Ser- |
| Lys-Ala-Pro-Pro-Pro-Ser-Leu-Pro-Ser-Pro-Ser- |
| Arg-Leu-Pro-Gly-Pro-Ser-Asp-Thr-Pro-Ile-Leu- |
| Pro-Gln-Pro-Pro-Pro-Pro-Pro-Pro-Cys |
| |
|---|
| Structure (XI) |
| Asp-Asp-Pro-Arg-Phe-Gln-Asp-Ser-Ser-Ser-Ser- |
| Lys-Ala-Pro-Pro-Pro-Ser-Leu-Pro-Ser-Pro-Ser- |
| Arg-Leu-Pro-Gly-Pro-Ser-Asp-Thr-Pro-Ile-Leu- |
| Pro-Gln-Cys |
| |
|---|
| Structure (XII) |
| Thr-Cys-Asp-Asp-Pro-Arg-Phe-Gln-Asp-Ser-Ser. |
| Ser-Ser-Lys-Ala-Pro-Pro-Pro-Set-Leu-Pro-Ser- |
| Pro-Ser-Arg-Leu-Pro-Gly-Pro-Ser-Asp-Thr-Pro- |
| Ile-Leu-Pro-Gln |
| |
|---|
| Structure (XIII) |
| Asp-His-Pro-Leu-Thr-Aba-Asp-Asp-Pro-Asg-Phe- |
| Gln-Asp-Ser-Ser-Ser-Ser-Lys-Ala-Pro-Pro- |
| Ser-Leu-Pro-Ser-Pro-Ser-Arg-Lev-Pro-Gly-Pro- |
| Ser-Asp-Thr-Pro-Ile-Leu-Pro-Gln-Cys |
| |
|---|
| Structure (XIV) |
| Cys-Pro-Pro-Pro-Pro-Pro-Pro-Pro-Asp-Asp-Pro- |
| Arg-Phe-Gln-Asp-Ser-Ser-Ser-Ser-Lys-Ala-Pro- |
| Pro-Pro-Ser-Leu-Pro-Ser-Pro-Ser-Arg-Leu-Pro- |
| Gdy-Pro-Ser-Asp-Thr-Pro-Ile-Leu-Pro-Gln |
b) un fragment de polypeptide antigénique analogue aux acides aminés 38 à 57 de la sous-unité bêta de la gonadotropine chorionique humaine choisi dans le groupe constitué par les structures XXV, XXVI, XXVII, XXVIII, XXIX, XXX et XXXI
| (Structure XXV) |
|---|
| Cys-Pro-Ser-Met-Lys-Arg-Val-Leu-Pro-Val-Ile-Leu- |
| Pro-Pro-Met-Pro-Gln-Arg-Val-Cys; |
| |
|---|
| (Structure XXVI) |
| Cys-Pro-Thr-Met-Met-Arg-Val-Leu-Gln-Ala-Val-Leu- |
| Pro-Pro-Leu-Pro-Gln-Val-Val-Cys; |
| |
|---|
| (Structure XXVII) |
| Cys-Pro-Thr-Met-Thr-Arg-Val-Leu-Gln-Gly-Val-Leu- |
| Pro-Ala-Leu-Pro-Gln-Val-Val-Cys; |
| |
|---|
| (Structure XXVIII) |
| Cys-Tyr-Thr-Arg-Asp-Leu-Val-Tyr-Lys-Asn-Pro-Ala- |
| Arg-Pro-Lys-Ile-Gln-Lys-Thr-Cys, |
| |
|---|
| (Structure XXIX) |
| Cys-Tyr-Thr-Arg-Asp-Leu-Val-Tyr-Lys-Asp-Pro-Ala- |
| Arg-Pro-Lys-Ile-Gln-Lys-Thr-Cys; |
| |
|---|
| (Structure XXX) |
| Cys-Pro-Ser-Met-Val-Arg-Val-Thr-Pro-Ala-Ala-Leu- |
| Pro-Ala-Ile-Pro-Gln-Pro-Val-Cys; |
| |
|---|
| (Structure XXXI) |
| Cys-Met-Thr-Arg-Asp-Ile-Asp-Gly-Lys-Leu-Phe-Leu- |
| Pro-(Lys-Tyr)-Ala-Leu-Ser-Gln-Asp-Val-Cys. |
dans lequel les deux résidus cystéine sont liés pour former une liaison disulfure,
lesdits fragments de polypeptides antigéniques a) et b) étant modifiés par une conjugaison au niveau de l'extrémité N-terminale ou de l'extrémité C-terminale avec un vecteur constitué par un peptide espaceur contenant de 2 à 8 acides aminés et soit une anatoxine diphtérique soit un fragment de peptide choisi dans le groupe de séquences d'épitope constitué par
a) les acides aminés 580 à 599 de l'anatoxine tétanique
b) les acides aminés 830 à 844 de l'anatoxine tétanique
c) les acides aminés 916 à 932 de l'anatoxine tétanique
d) les acides aminés 947 à 967 de l'anatoxine tétanique
e) les acides aminés 288 à 302 de la protéine du virus de la rougeole,
f) les acides aminés 16 à 33 de la protéine du virus de l'hépatite B
g) les acides aminés 317 à 336 de la protéine CSP du paludisme et
h) des mélanges desdits épitopes a) à g), et
du squalane et/ou du squalène en tant qu'un agent émulsifiant et un adjuvant,
dans lequel lesdits polypeptides antigéniques sont combinés pour former un peptide de fusion, dans lequel lesdits polypeptides antigéniques a) et b) sont séparément conjugués au vecteur, ou dans lequel lesdits polypeptides antigéniques a) et b) sont conjugués à différents vecteurs et utilisés en tant qu'un mélange.

2. Vaccin selon la revendication 1, dans lequel l'anatoxine diphtérique est un produit de réaction de l'anatoxine diphtérique et de l'éthylènediamine.

3. Vaccin selon les revendications 1 et 2, dans lequel le conjugué antigénique comprend de 20 à 30 polypeptides pour 10⁵ daltons de l'anatoxine diphtérique modifiée.

4. Vaccins selon l'une quelconque des revendications 1 à 3, contenant en outre du monooléate de mannide et du monostéarate d'aluminium.

5. Vaccin selon la revendication 4, comprenant, en poids, de 35 à 45 % de squalène, de 35 à 45 % de squalane, de 6 à 16 % de monooléate de mannide et de 1 à 5 % de monostéarate d'aluminium.

6. Vaccin selon l'une quelconque des revendications 1 à 5, dans lequel l'adjuvant est la N-acétyl-D-glucosamine-3-yl-acétyl-L-ala-D-isoglutamine (nor muramyl dipeptide).

7. Vaccin selon l'une quelconque des revendications 1 à 6, comprenant de 0,2 à 1,0 mg de l'adjuvant par mL de vaccin.

8. Vaccin selon l'une quelconque des revendications 1 à 7, dans lequel la teneur en conjugué antigénique est de 0,5 à 2,0 mg par mL de vaccin.

9. Vaccin selon l'une quelconque des revendications 1 à 8, dans lequel le vaccin comprend des quantités égales de la phase aqueuse et de l'agent émulsifiant.

10. Vaccin selon l'une quelconque des revendications 1 à 9, dans lequel la phase aqueuse est une solution saline tamponnée au phosphate.

11. Utilisation du vaccin selon l'une quelconque des revendications 1 à 10, pour la préparation d'un médicament destiné au traitement du cancer du côlon.
